(19) Europäisches Patentamt
European Patent Office
Office européen des brevets

(11) **EP 2 694 958 B1**

(12) # EUROPEAN PATENT SPECIFICATION

(45) Date of publication and mention of the grant of the patent:
**07.10.2020 Bulletin 2020/41**

(21) Application number: **12712281.0**

(22) Date of filing: **05.04.2012**

(51) Int Cl.:
***G01N 31/22*** *(2006.01)* ***C07H 23/00*** *(2006.01)*
***G01N 21/77*** *(2006.01)* ***G01N 21/78*** *(2006.01)*

(86) International application number:
**PCT/EP2012/056331**

(87) International publication number:
**WO 2012/136793 (11.10.2012 Gazette 2012/41)**

(54) **OPTICAL DETECTION OF CYANIDES, METHOD, KIT AND DEVICE**

OPTISCHER NACHWEIS VON CYANIDEN, VERFAHREN, KIT UND VORRICHTUNG

DETECTION OPTIQUE DE CYANIDES, PROCEDE, KIT ET DISPOSITIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.04.2011 EP 11161228**
**26.08.2011 EP 11179084**
**15.12.2011 EP 11193853**

(43) Date of publication of application:
**12.02.2014 Bulletin 2014/07**

(73) Proprietor: **CyanoGuard AG**
**8952 Schlieren (CH)**

(72) Inventors:
- **ZELDER, Felix**
  **CH-8006 Zürich (CH)**
- **MÄNNEL-CROISÉ, Christine**
  **CH-5702 Niederlenz (CH)**

(74) Representative: **McStea, John Anthony**
**Teemacs GmbH**
**Kantonsstrasse 7**
**CH-4416 Bubendorf (CH)**

(56) References cited:
**WO-A2-2006/009874** **WO-A2-2011/116006**
**DE-A1- 19 608 808** **US-A1- 2009 291 144**

- **SYLVIA. DAUNERT ET AL: "Anion-selective electrodes based on a hydrophobic vitamin B12 derivative", ANALYTICAL CHEMISTRY, vol. 61, no. 5, 1 March 1989 (1989-03-01), pages 499-503, XP55032039, ISSN: 0003-2700, DOI: 10.1021/ac00180a025**
- **William C. Blackledge ET AL: "New Facile Method to Measure Cyanide in Blood", Analytical Chemistry, vol. 82, no. 10, 15 May 2010 (2010-05-15), pages 4216-4221, XP055329153, US ISSN: 0003-2700, DOI: 10.1021/ac100519z**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 694 958 B1

## Description

### Field of the Invention

The present invention generally relates to a method for selective detection of cyanide. Moreover, the invention relates to a kit for selective detection of cyanide and to uses thereof. Furthermore, the invention relates to a device comprising modified silica that contains corrinoid compounds.

### Background of the Invention

Cyanide is highly toxic to humans and most animals because of inactivation of cytochrome C oxidase. It is widely used for important industrial applications and is stored in form of cyanogenic glycosides in more than 2'000 plants such as bamboo, flax seed or cassava (*Manihot esculenta* Crantz). The latter represents common foodstuff for hundred of millions of people in the developing countries. Inadequately processed cassava products might cause severe acute and chronic health problems. In view of all the above, there is considerable interest in sensitive and selective methods for detecting cyanide in relevant environments such as drinking water, foodstuffs and industrial wastewaters. Several methods for detecting cyanide are known in the art. Recently, numerous novel reagents for the colorimetric detection of cyanide have been reported. In particular, DE 196 08 808 A1 discloses a colorimetric detection principle based on a positively charged cobinic acid amide compound loaded on an cation exchanger acting as an indicator for cyanide anions or hydrogen cyanide.

The optical detection of cyanide with corrinoids under homogenous conditions has also been demonstrated. See, e.g.: Zelder, F. H. Inorg. Chem. 2008, 47, 1264-1266; Männel-Croise, C.; Zelder, F. Inorg. Chem. 2009, 48, 1272-1274.

Nevertheless, it would be desirable to have further cyanide sensors with even higher sensitivity. In particular, it would be desirable to have a method for visual detection of cyanide below the guideline value of cyanide in drinking water of the World Health Organization (WHO; 0.05 mg/ l) (i), the simultaneous visual detection of cyanide and thiocyanate (ii) as well potential applications for the detection of cyanide in foodstuff as well as HCN in cigarette smoke (iii).

Solid-phase extraction (SPE) has generally proven very useful for sample preparation in modern analytical chemistry. The method includes sample preconcentration and prepuri-fication for subsequent further analysis by chromatography, mass spectrometry and other techniques. Applications in almost all areas of chemical analysis including pharmaceutical research, food analysis and drinking water control have been reported. Colorimetric solid phase extraction (C-SPE) describes the concentration and quantification of an analyte directly on a solid support with diffuse reflectance UV-Vis (DRUV-Vis) spectroscopy and has been successfully applied for the quantification of analytes such as $I^-/I_2$, formaldehyde, Ni(II), Mo(VI), Pb(II) or As(V). See, e.g.: Arena, M. P.; Porter, M. D.; Fritz, J. S. Anal. Chem. 2002, 74, 185-190.

### Summary of the Invention

According to one aspect of the invention, there is provided a method for selective detection of cyanide in a liquid sample, the method comprising determining a color change of a corrinoid compound caused upon direct contact with said sample, wherein said corrinoid compound is selected from the group consisting of (see below for definitions):

- a cobalamine according to formula (**I**),
  wherein X is CN, $OH_2$, OH, Hal (Cl, Br, I) or $CH_3$
- a cobyrinic acid hepta C1-4 alkyl ester derivative (**II**) wherein X is CN, $OH_2$, OH, Hal (Cl, Br, I) or $CH_3$
- a cobyrinic acid (**III**) wherein X is CN, $OH_2$, OH, Hal (Cl, Br, I) or $CH_3$ and
- a cobinamide (**IV**) wherein X is CN, $OH_2$, OH, Hal (Cl, Br, I) or $CH_3$

wherein said corrinoid compound is adsorbed on a non-polar solid $C_4$ - $C_{18}$ silica phase.

In the above, the compounds of formula (**I**) are as follows:

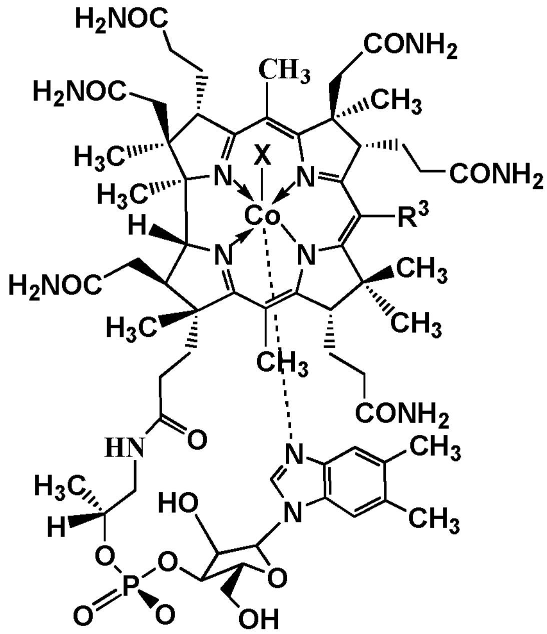

**I:** **X = CN, $OH_2$, OH, Hal (Cl-, Br-, I-) or $CH_3$,**
**$R^3$= H, Hal (Cl-, Br-, I-), $CF_3$, -alkyl**

and the compounds (**II**), (**III**) and (**IV**) are as follows:

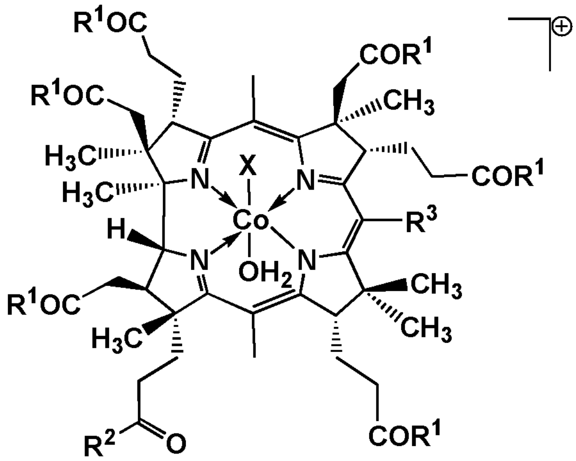

**II:** **$R^1$= $R^2$ = $OC_nH_{2n+1}$,**
**III:** **$R^1$= $R^2$ = OH**
**IV:** **$R^1$= $NH_2$, $R^2$ = $NHCH_2CH(CH_3)OH$,**
**II-IV: X = CN, $OH_2$, OH, Hal (Cl-, Br-, I-) or $CH_3$,**
**$R^3$= H, Hal (Cl-, Br-, I-), $CF_3$, -alkyl**

Surprisingly, it was found that by adsorbing the above mentioned corrinoid compounds as chemosensors on a solid $C_4$-$C_{18}$ silica phase it is possible to realize a highly sensitive and convenient colorimetric detection system for cyanide.

The chemosensors are either commercially available or readily synthesized from commercially available precursors and have been adsorbed on the solid phase without further chemical modifications.

It shall be understood that for any of the above compounds that are chiral, it will generally be acceptable to have a mixture of diastereomers as far as the principle of this invention is concerned. On the other hand, certain compounds will naturally be produced by bacteria in a specific diastereomeric form, which thus will be available more

readily than another diastereomeric form.

The term "hepta C1-4 alkyl ester" shall be understood as any linear or branched alkyl ester with up to 4 carbon atoms.

The solid phase acting as an adsorber may be any suitable solid with a large surface area, particularly a material customarily used for chromatography applications. In view of the colorimetric detection principle, the solid phase should be basically white.

According to a preferred embodiment, the corrinoid compound is a cobalamine compound, particularly one of the vitamin B12 vitamers β-cyanocobalamine or β-aquocobalamine or β-hydroxocobalamine. As will generally be known, the latter two compounds are produced by certain bacteria and are converted to β-cyanocobalamine in the process of being purified in activated charcoal. Therefore, β-cyanocobalamine (1) is considered to be a most preferred compound for the purpose of this invention.

Systems with immobilized B 12 as the chemosensor show high sensitivity combined with excellent selectivity towards cyanide. Without being bound by theory, it appears that the up to 130 times higher sensitivity of immobilized B 12 towards cyanide compared to the optical detection in water results mainly from two different effects: (i) the extraction and concentration of cyanide from the sample solution, as well as (ii) the altered coordination geometry of the immobilized chemosensor within the hydrophobic solid-phase. The latter effect makes cyanide binding more likely.

According to another embodiment, the corrinoid compound is one of several so-called "incomplete" corrinoids such as a cyano-aqua-cobyrinic acid hepta C1-4 alkyl ester (**2**), cyano-aqua-cobyrinic acid (**3**) or cyano-aqua-cobinamide (**4**). These B 12 derivatives lacking the nucleotide at the f-side chain were found to be even more sensitive and allowed the visual detection of cyanide below the guideline value for drinking water of the World Health Organisation (WHO; 0.05 mg/l).

According to one embodiment, the method according to this invention is applied for detecting cyanide ions in an aqueous medium.

By virtue of the high selectivity and specificity of the colorimetric reactions, the method according to the present invention may also be applied with samples that also contain further species such as thiocyanate ions and/or isocyanate ions and/or iodide ions, which often cause problems in other cyanide detection methods.

According to a further embodiment, the method of detecting cyanide further comprises the step of also detecting any thiocyanate ions and/or isocyanate ions and/or iodide ions that are present in the sample.

It will be understood that the colorimetric detection step may be realized in several ways. Under certain circumstances it is possible to detect the presence or absence of cyanide by simple visual inspection, i.e. by checking for a discernible color effect. In a further embodiment, the detection is carried out by measurements of diffuse reflectance in the UV/visible spectral range. Further embodiments, which are particularly useful for an automated and fast screening, comprise the use of a digital camera with red/green/blue (RGB) channel encoding.

According to a further embodiment, the method of detecting cyanide further comprises the step of removing at least one interfering colored component contained in the sample. This is particularly helpful for removing green colored components that may be present in liquid samples containing raw extracts from plants.

In many embodiments the direct contact between the liquid sample and the corrinoid compound adsorbed on a solid phase is achieved by transporting the liquid sample to a suitable solid carrier onto which the appropriate corrinoid compound has previously been adsorbed. In another embodiment, however, the liquid sample and the corrinoid compound are brought together in a liquid phase, the latter usually containing an appropriate solvent, and this liquid phase is then transported to a suitable carrier, to which the corrinoid compound will be adsorbed together with the sample species. This latter method is particularly useful for detection of cyanide in a liquid blood sample.

A kit for selective detection of cyanide in a sample comprises at least one first colorimetric solid phase extraction device, said first device comprising a non-polar solid $C_4$-$C_{18}$ silica phase loaded with a first corrinoid compound selected from the group consisting of:

- a cobalamine according to formula (**I**),
  wherein X is CN, $OH_2$, OH, Hal (Cl, Br, I) or $CH_3$
- a cobyrinic acid hepta C1-4 alkyl ester derivative (**II**) wherein X is CN, $OH_2$, OH, Hal (Cl, Br, I) or $CH_3$
- a cobyrinic acid (**III**) wherein X is CN, $OH_2$, OH, Hal (Cl, Br, I) or $CH_3$ and
- a cobinamide (**IV**) wherein X is CN, $OH_2$, OH, Hal (Cl, Br, I) or $CH_3$ means for passing a gaseous or liquid sample over or across said device, and means for detecting a color change of said first corrinoid compound.

In a further embodiment, the kit as defined above further comprises at least one further colorimetric solid phase extraction device, said further device comprising a non-polar solid phase loaded with a further corrinoid compound selected from the group consisting of

- a cobalamine according to formula (**I**),

wherein X is CN, $OH_2$, OH, Hal (Cl, Br, I) or $CH_3$

- a cobyrinic acid hepta C1-4 alkyl ester derivative (**II**) wherein X is CN, $OH_2$, OH, Hal (Cl, Br, I) or $CH_3$
- a cobyrinic acid (**III**) wherein X is CN, $OH_2$, OH, Hal (Cl, Br, I) or $CH_3$ and
- a cobinamide (**IV**) wherein X is CN, $OH_2$, OH, Hal (Cl, Br, I) or $CH_3$

with the provision that said first and said second corrinoid compounds are different, the kit further comprising means for passing a gaseous or liquid sample sequentially over or across said first device and said further device, and means for detecting a color change of each one of said corrinoid compounds. Such a kit may be used for the simultaneous visual detection of cyanide in conjunction with thiocyanate ions and/or isocyanate ions and/or iodide ions and will thus be called "multi-detection kit".

In general, such a multi-detection kit will also include an adsorber stage that removes one of the detected species, e.g. cyanide, from the sample after it has passed the respective detection stage. Such adsorber stage will thus generally be arranged between two detection stages.

Moreover, it may be advantageous if the detection kit further comprises at least one clearing device for removing interfering components contained in a liquid sample, said clearing device arranged before said first colorimetric solid phase extraction device.

The kits of the present invention are particularly useful for the visual detection of cyanides in drinking water or foodstuff or industrial wastewaters.

As a separate application, the kits of the present invention may be used for the visual detection of cyanide and/or hydrogen cyanide in a colored liquid phase. It will be understood that, e.g. in an aqueous phase, the relative amounts of cyanide ions and hydrogen cyanide will depend on the pH-value.

According to still another aspect of the invention, there is provided a device for removing hydrogen cyanide from tobacco smoke, the device comprising an adsorption medium containing a corrinoid compound adsorbed on a solid phase substrate. Basically, such a device could be incorporated in the filter portion of a cigarette.

According to still another aspect of the invention, there is provided a device for removing hydrogen cyanide from a subject's blood, the device comprising an adsorption medium containing a corrinoid compound adsorbed on a solid phase substrate. In particular, such a device could be incorporated in a hemodialysis apparatus. It will be understood that the underlying principle could also be applied by admixing the corrinoid compound into the subject's blood and subsequently adsorbing the corrinoid compound together with the cyanide by adsorption on a solid phase substrate.

## Brief description of the drawings

The above mentioned and other features and objects of this invention and the manner of achieving them will become more apparent and this invention itself will be better understood by reference to the following description of various embodiments of this invention taken in conjunction with the accompanying drawings, which show the following:

**Figure 1** **a)** Experimental set-up for the use of C18ec columns for the detection of cyanide in aqueous solution.
**b)** Experimental set-up for the detection of HCN in the smoke of a cigarette with C18ec cartridges.

**Figure 2** **a)** (From left to right) Side and top views on $\mathbf{1_{SP}}$ (20 nmol; red coloured ring) and after passing solution of $CN^-$ (0.26 mg/l, 5 ml, [Ches] = 20 mM, pH 9.5) through the column.
**b)** Corresponding DRUV-vis spectra.

**Figure 3** Absorption spectrum of 1 (40 μM in water; [Hepes] = 20 mM; pH 7.0; *blue),* DRUV-vis spectra of $\mathbf{1_{SP}}$ (20 nmol; *red*) and $\mathbf{1_{Silica}}$ (20 nmol; *black*) ([Hepes] = 20 mM; pH 7.5).

**Figure 4** **a)** DRUV-vis spectra of $\mathbf{2_{SP}}$ (20 nmol) and $\mathbf{2_{SP}}$ (20 nmol) after passing solutions (5 ml, [Ches] = 20 mM, pH 9.5) of $CN^-$ (0.039 mg/l, 0.078 mg/l, 0.130 mg/l and 0.182 mg/l) through the column.
**b)** Corresponding CIELab values: L*, a*, b*, and ΔE. ΔL*, Δa*, Δb* = ± 0.1, (ref. = reference).

**Figure 5** **a)** (*From left to right*) Top and side views on $\mathbf{2_{SP}}$ (20 nmol, orange coloured ring) and $\mathbf{2_{SP}}$ (20 nmol) after passing solutions (5 ml, [Ches] = 20 mM, pH 9.5) of $CN^-$ (0.039 mg/l, 0.078 mg/l and 0.182 mg/l) through the column.
**b)** Calibration curve for $CN^-$ at 583 nm and pH 7.5 (a) or pH 9.5 (b) of $\mathbf{2_{SP}\text{-}CN}$.

**Figure 6** **a)** *(from left to right):* Side and top view on $\mathbf{2_{SP}}$ (20 nmol, orange coloured ring) and $\mathbf{2_{SP}}$ (20 nmol) after passing solutions (5 ml, [Hepes] = 20 mM, pH 7.5) of $CN^-$ (0.039 mg/l, 0.078 mg/l and 0.182 mg/l) through the column.

**b)** Corresponding CIELab values: L*, a*, b*, and ΔE. ΔL*, Δa*, Δb* = ± 0.1, (ref. = reference).

**Figure 7** DRUV- vis spectra of $\mathbf{3_{SP}}$ (*dashed line,* $\lambda_{max}$= 525 nm) and $\mathbf{3_{SP}}$**-CN** (*solid line,* $\lambda_{max}$= 578 nm) after passing a solution (1 ml; [Ches] = 20 mM, pH 9.5) of a cassava extract through the filter.

**Figure 8** **a)** DRUV- vis spectrum of $\mathbf{1_{SP}}$ (*dashed line,* $\lambda_{max}$= 532 nm) and $\mathbf{1_{SP}}$ after passing the smoke of a cigarette through the C18ec-cartridge (*solid line,* $\mathbf{1_{SP}}$**-CN**, $\lambda_{shoulder}$= 579 nm).
**b)** DRUV- vis spectrum of $\mathbf{2_{SP}}$ (*dashed line,* $\lambda_{max}$= 525 nm) and $\mathbf{2_{SP}}$ after passing the smoke of a cigarette through the C18ec-cartridge (*solid line,* $\mathbf{2_{SP}}$**-CN,** λ= 578 nm).

**Figure 9** UV- vis spectra of **1**, **2**, **1+ CN$^-$** and **1+ SCN, 2-CN, 2-SCN, 2-CN+ 2-SCN.**

**a)** UV-Vis spectra of **2** (40 μM in water; [Ches] = 20 mM; pH 9.0) and after addition of $CN^-$ (20 μM; [Ches] = 20 mM; pH 9.0), $SCN^-$ (5 mM; [Ches] = 20 mM; pH 9.0), and a mixture of $CN^-$ and $SCN^-$ (20 μM and 5 mM; [Ches] = 20 mM; pH 9.0),
**b)** UV-Vis spectra of 1 (40 μM in water; [Ches] = 20 mM; pH 9.0) and after addition of $CN^-$ (20 μM; [Ches] = 20 mM; pH 9.0) and $SCN^-$ (5 mM; [Ches] = 20 mM; pH 9.0).

**Figure 10** Construction kit for simultaneous anion detection consisting of three C18ec cartridges impregnated with $\mathbf{1_{SP}}$ and $\mathbf{2_{SP}}$. Column left: Cartridge 1) cyanide detection zone: $\mathbf{1_{SP}}$ (50 nmol). Cartridge 2) cyanide extraction zone: $\mathbf{1_{SP}}$ (200 nmol (*top*)/ 50 nmol (*bottom*). Cartridge 3) thiocyanate detection zone: $\mathbf{2_{SP}}$ (50 nmol). Column right: after passing a mixture (8 ml, [Ches] = 20 mM, pH 9.5) of $CN^-$ (0.52 mg/l) and $SCN^-$ (0.13 g/l) through the cartridges shown on the left.

**Figure 11** $\mathbf{3_{TLC}}$ before after being dipped into sample solutions with increasing concentration of cyanide. *(From left to right):* $\mathbf{3_{TLC}}$ and $\mathbf{3_{TLC}}$ after being dipped for 10 minutes into sample solutions ([Ches] = 20 mM, pH 9.5) with increasing concentrations of cyanide (0, 0.26 mg/l, 0.52 mg/l , 1.04mg/l , 1.56 mg/l and 2.08 mg/l).

**Figure 12** Detection of cyanide in the crude sample of a green colored cassava leaf:

**a)** Experimental set-up before injecting green colored sample;
**b)** Experimental set-up after passing green colored sample through the device;
**c)** DRUV-vis spectra of $\mathbf{1_{SP}}$ before (*black*) and after (*red*) passing the solution through the device.

**Figure 13** **a)** DRUV-Vis spectra of $\mathbf{2_{SP}}$ (red) and $\mathbf{2_{SP}}$**-CN** (black) upon blood spiked with cyanide passed the column (c(CN-)blood= 91 μM;
**b)** Experimental set-up for the detection of cyanide in diluted fresh blood (pH 9.5, Ches 20 mM).

## Detailed description of the invention

### *Experimental Section*

### Materials. General information

Potassium cyanide, Vitamin $B_{12}$ (**1**), dicyano-cobyrinic acid heptamethylester (**2-CN**), dicyano-cobinamide (**4-CN**), Ches and Hepes were obtained from Fluka (Buchs, CH) or Sigma Aldrich. Cyano-aqua-cobyrinic acid heptamethylester **2,** cyano-aqua-cobyrinic acid **3** and cyano-aqua-cobinamide **4** were synthesized as pairs of diastereomers from their corresponding dicyano-forms through non-selective displacement of either the (upper) β- or the (lower) α-cyanide as described elsewhere. Compounds **2** and **2-CN** are susceptible for hydrolysis at the ester side chains, especially at basic pH. Therefore also compounds with partially hydrolysed side chains can be applied. KCN stock solutions ($10^{-3}$ M) were prepared freshly before use. The desired pH values of the stock solutions of the buffers Ches (0.1 M; pH 9.5) and Hepes (0.1 M; pH 7.5) were adjusted by the addition of either a solution of 2 N NaOH or 1 N HCl. All measurements were performed at a final buffer concentration of 20 mM.

Chromabond C18ec polypropylene columns (100 mg) and Chromafix C18ec cartridges (270 mg) were obtained from Machery-Nagel AG Schweiz. In this paper we will use the terms C18ec columns for the former, C18ec cartridges for the latter, and C18ec material for both types, respectively. Polygram polyester sheets Alox N and Octadecyl-modified nano silica layers RP-18W were also obtained from Machery-Nagel AG Schweiz. Solvents of HPLC grade of highest purity and doubly distilled water were used.

Waxed cassava roots (*Manihot esculenta* Crantz), imported from Costa Rica, and cigarettes were purchased at local supermarkets in Zurich, Switzerland. Leaves from *Manihot esculenta* Crantz were a generous gift from the botanical garden of the university of Zurich.

**C-SPE-method**

*General.* The colorimetric solid phase extraction (CSPE) devise consisted of a 10 ml syringe that was either connected directly or via a Chromabond adapter PP (Macherey-Nagel) to C18ec columns or C18ec cartridges (**Figure 1a**). The sample solution was pressed with a rate of approximately 1 ml/ min for $\mathbf{1_{SP}}$ (SP for "solid phase") and 2 ml/ min for $\mathbf{2_{SP}}$- $\mathbf{4_{SP}}$ through the C18ec material. The procedure is exemplified for chemosensor 1.

*1. Conditioning.* The C18ec filter was washed with methanol (0.5 ml) and water (10 ml).
*2. Adsorption of Chemosensor.* The chemosensor **1** was adsorbed on the top of the C18ec material while an aqueous solution of **1** (0.5 ml; 40 μM) was passed through the filter material. The immobilized chemosensor $\mathbf{1_{SP}}$ (SP for "solid phase") was visible as a red coloured-ring (height ~ 1 mm). *3. Analysis.* A sample solution was passed through the filter. Detection with $\mathbf{1_{SP}}$ was indicated by color change. 4. *Washing.* The solid support was washed with water (10 ml). *5. Regeneration.* $\mathbf{1_{SP}}$ was regenerated from $\mathbf{1_{SP}}$**-CN** while passing 0.1 -1 % of aqueous citric acid, acetic acid or trifluoroacetic acid (3 ml) and water (20 ml) through the filter.

**Test strips**

*Preparation.* Polygram polyester sheets Alox N and Octadecyl-modified nano silica layers RP-18W from Machery-Nagel AG Schweiz were used as test strips. The Alox-sheets and the RP-18W- plates were cut in test stripes of diameters of 15 x 5 mm for the former and 12 x 15 mm for the latter, respectively. The test strips were dipped (2 sec) into an aqueous solution of **3** (1 mM) for Alox N and of **2** (1 mM) for RP-18W. Afterwards they were dried for 10 min at 60 °C and then for 1 h at 22 °C.

*Analysis.* The impregnated test strips were dipped for at least 10 minutes into the analyte solution. In the presence of cyanide, a color change from orange to violet was observed within this time. The color of the test strips remained stable for at least 8 h.

**Spectroscopic measurements**

UV-vis spectra were measured at T = 21 ±1°C with a Cary 50 spectrometer using quartz cells with a path length of 1 cm. DRUV-vis spectra were recorded on a Perkin-Elmer Lambda 50 spectrometer equipped with an integrating sphere setup (diameter 110 mm) and pure $MgSO_4$ as reference material.

**Sample preparation for diffuse reflectance (DRUV-vis) spectroscopy**

For DRUV-vis measurements of immobilized chemosensors and immobilized chemosensor-analyte complexes, the cartridges or columns were opened under greatest care. The top layer containing the immobilized chemosensor-analyte complex was removed from the cartridges or columns, dried 30 min on air) and used for DRUV-vis measurements.

**Quantification/ Kubelka Munk**

The percentage of reflectance (R) was measured with respect to silica C18ec or to an unmodified Alox-plate as standard white. The Kubelka Munk equation gives the relation between the percentage of reflectance (R) and the concentration of the analyte under assumption of a constant molar absorptivity (ε) and scattering coefficient (s) for a given wavelength:

$$F(R) = (1 - R)^2 / 2R$$

$$F(R) = \varepsilon \cdot C / s.$$

**CIELab measurements**

A handheld spectrophotometer CM-2900d from Minolta was used to measure the differences of color in the

CIELAB-system with Specular Component Excluded (SCE). All values are averaged from at least 8 measurements. The differences in color ΔE have bee estimated according to:

$$\Delta E = [(\Delta L^*)^2 + (\Delta a^*)^2 + (\Delta b^*)^2]^{1/2}.$$

For the description of color differences the major sphere of ΔE was used.

**Detection of endogenous cyanide in foodstuff with C-SPE**

Preparation of a cassava root extract was performed as described earlier. The crude solution (20 μl) was diluted with water (980 μl; pH 9.5 [Ches] = 0.1 M). Afterward this sample solution was used for C-SPE.

**Detection of HCN in cigarette smoke with C-SPE**

To detect hydrogen cyanide (HCN) in the smoke of a cigarette, two C18ec cartridges were connected as shown in **Figure 1b.** The first cartridge was used to remove tar from the smoke; the second containing $\mathbf{1_{SP}}$ or $\mathbf{2_{SP}}$ was applied to detect HCN.

A vacuum (400 mbar) was applied to pull the smoke of the cigarette through the cartridges. The top layer of cartridge 2 containing the immobilized chemosensor-analyte complex was carefully removed, dried (10 min at 45°C and 30 min on air) and used for DRUV-vis measurements.

**C-SPE with B12 and B12-derivatives immobilized on polymeric columns and discs containing C18 material**

Empore™ SPE material (EC-SPE, 1ml, product number 4115) with C18-SD was also useful for the visual detection of cyanide. In particular, cyanide was also detected using a self-made filter holder device and polymeric discs containing $\mathbf{1_{SP}}$ and $\mathbf{2_{SP}}$ on C18 filter material (EmporeTM, product number 2215). EmporeTM SPE colums (EC-SPE, 1ml, product number 4115) and EmporeTM extraction discs (C18, diameter of 47 mm, product number 2215) were obtained from 3M Deutschland GmbH. Discs of smaller diameter (4 mm) were made from EmporeTM extraction discs using a hole puncher. Immobilization of **1** and **2** onto the polymer and detection of cyanide was performed with $\mathbf{1_{SP}}$ and $\mathbf{2_{SP}}$ as described for the C18ec-columns. These disks were inserted into a filter holder device that was constructed in the mechanical workshop of the Institute of Inorganic Chemistry of the University of Zurich.

***Results and Discussion***

**Colorimetric solid phase extraction (C-SPE) with vitamin B12**

The detection of cyanide with vitamin B12 (**1**) is based on the replacement of the intramolecularly bound dimethylbenzimidazole (Dmbz; B) base of **1** with cyanide to form a negatively charged 'base off dicyano-B12 species (**1-CN; Scheme 1,** equilibrium I). This reaction is accompanied by a red shift of the absorption maxima ($\Delta\lambda_{max}$ = 45 nm) and a color change from red to violet.

**Scheme 1.** Proposed mechanism for the binding of cyanide to the `base on` form of **1** to yield `base off` **1-CN** in water (equilibrium I), partial dissociation of the Dmbz base (B) of **1** on silica-C18ec to form $\mathbf{1_{SP}}$ as a mixture of its `base on` and `base off` forms (equilibrium II). Binding of cyanide to $\mathbf{1_{SP}}$ to form $\mathbf{1_{SP}}$**-CN** during C-SPE (equilibrium III):

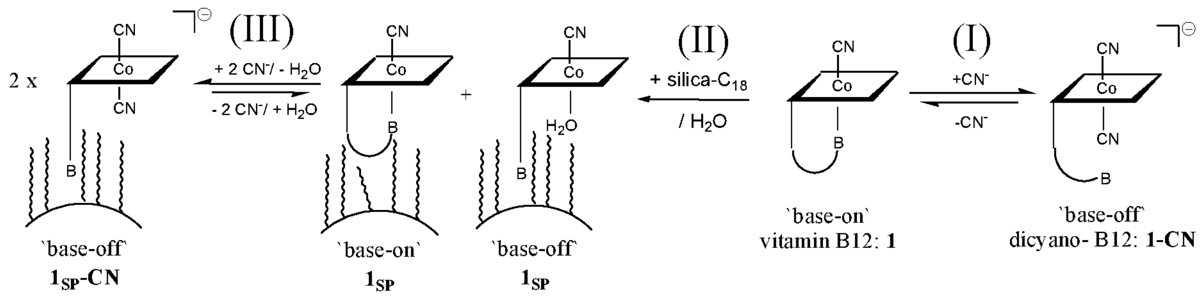


To apply C-SPE for the detection of cyanide, **1** was immobilized on a commercially available chromabond C18ec column as described in the experimental section. Thereby **1** was concentrated as $\mathbf{1_{SP}}$ (SP for "solid phase") on the surface of the white C18 material, visible as a red colored ring (**Figure 2a**). The strong intramolecular coordination of the Dmbz base (B) to the cobalt center of 1 in water is expressed by a low $pK_{base\text{-}off}$ of 0.1. Compound **1** therefore exists in water exclusively in its 'base on' form that is easily identifiable by its absorption spectrum (**Figure 3**). The DRUV-vis spectrum of immobilized $\mathbf{1_{SP}}$ on the solid support shows significant differences compared to the absorption spectrum of 1 in water with a blue shift of 18 nm (**Figure 3**). This shift is indicative of differences in the coordination environment of the cobalt(III) center.

The DRUV-vis spectrum of $\mathbf{1_{SP}}$ looks similar to the absorption spectrum of a mixture of both, the 'base on' and 'base off forms of **1.** 'Base off **1** has been earlier observed for a supramolecular **1**-cucurbit[7]uril complex, in which the α-(lower side) coordinated Dmbz base of **1** has been encapsulated into the hydrophobic host.

In accordance with these studies, we assume partial dissociation of the Dmbz base of **1** from the cobalt center and its binding within the hydrophobic $C_{18}$-chains of the solid-phase (**Scheme 1**, equilibrium II). This behavior reassembles the binding of cobalamines (Cbl) in their 'base off form in certain protein-Cbl and nucleic acids-Cbl complexes. 'Base off $\mathbf{1_{SP}}$ contains a cobalt(III)-bound water at the α-side (lower side) of the macro-cycle (**Scheme 1**, equilibrium II). Evidence of the proposed binding mode of $\mathbf{1_{SP}}$ was obtained from comparison with a reflectance spectrum of **1** adsorbed as $\mathbf{1_{Silica}}$ on the surface of unmodified silica material. In this case, the DRUV-vis spectrum of $\mathbf{1_{Silica}}$ was different to that of $\mathbf{1_{SP}}$, but comparable to that of 'base on' **1** in water (**Figure 3**). Unmodified silica lacking the C18 chains was apparently not capable of stabilizing the 'base off form **of 1.**

When aqueous solutions of cyanide (0-1.04 mg/l) were passed through the C18ec material with $\mathbf{1_{SP}}$ at pH 7.5, the color of $\mathbf{1_{SP}}$ changed immediately from red to violet. This suggests the formation of immobilized $\mathbf{1_{SP}}$**-CN.** The colored $\mathbf{1_{SP}}$**-CN** complex at the top layer of the C18 matrix material was subsequently investigated with DRUV-vis spectroscopy. The reflection spectrum with a typical maximum of the γ-band at 583 nm is comparable to the absorption maxima of **1-CN** under homogenous conditions. Concentrations as low as 1.0 mg/l of cyanide could be detected by the 'naked-eye' at pH 7.5 when a 5 ml sample solution was passed through the column (Table 1, entry 3). This value represents a 130 times lower concentration (Table 1, S: Sensitivity) and 13 times lower quantity of cyanide per quantity of chemosensor compared to the detection of cyanide with **1** under homogenous aqueous conditions (Table 1, entries 1 *vs* 3).

**Table 1.** Comparison of chemosensors **1,** $\mathbf{1_{SP}}$, 2, and $\mathbf{2_{SP}}$ for the visual detection of cyanide.

| Entry | Chemose nsor | pH | $c_{(CN)}$ [mg/l][g] | $n_{(CN)}$ [nmol] | RQ[h] | F[i] | S[j] |
|---|---|---|---|---|---|---|---|
| 1[a], [b] | **1** | 7.5 | 130 | 5000 | 125 | - | - |
| 2[c] | **1** | 9.5 | 13 | 500 | 12.5 | - | - |
| 3[d] | $\mathbf{1_{SP}}$ | 7.5 | 1.0 | 200 | 10 | 13 | 130 |
| 4[e] | $\mathbf{1_{SP}}$ | 9.5 | 0.3 | 50 | 2.5 | 5 | 43 |
| 5[a], [f] | **2** | 7.5 | 0.3 | 10 | 0.25 | - | - |

(continued)

| Entry | Chemose nsor | pH | $c_{(CN)}$ [mg/l][g] | $n_{(CN)}$ [nmol] | RQ[h] | F[i] | S[j] |
|---|---|---|---|---|---|---|---|
| 6[c], [f] | **2** | 9.5 | 0.3 | 10 | 0.25 | - | - |
| 7[d] | $\mathbf{2_{SP}}$ | 7.5 | 0.04 | 7.5 | 0.375 | 0.7 | 7 |
| 8[e] | $\mathbf{2_{SP}}$ | 9.5 | 0.04 | 7.5 | 0.375 | 0.7 | 7 |

[a] [**1**] = [**2**] = 40 nmol, [Hepes] = 20 mM, V = 1ml. [b] (Zelder, F. H. *Inorg. Chem.* **2008,** *47*, 1264-1266). [c] [**1**] = [**2**] = 40 nmol; [Ches] = 20 mM, V = 1 ml. [d] [$\mathbf{1_{SP}}$] = [$\mathbf{2_{SP}}$] = 20 nmol, [Hepes] = 20 mM, V = 5 ml. [e] [$\mathbf{1_{SP}}$] = [$\mathbf{2_{SP}}$] = 20 nmol; [Ches] = 20 mM, V = 5 ml. [f] (Männel-Croise, C.; Zelder, F. *Inorg. Chem.* **2009,** *48*, 1272-1274). [g] > 10% over the minimum visual detectable concentration of cyanide [h] RQ: Ratio of quantities, RQ = $n_{[CN]}/ n_{[CS]}$, CS: chemosensor [i] F: hydrophobic effect, F = RQ/ $RQ_{SP}$. [j] S: Sensitivity, S = $\mathbf{c_{SP}}$ (**CN**$^-$)/ c(**CN**$^-$).

The latter effect (F) can be explained by the altered coordination geometry of the metal-based chemosensor within the hydrophobic solid-support that made the substitution of the Dmbz base with cyanide more likely. The enormous increase in sensitivity by applying C-SPE and B12 (**1**) has apparently two reasons (i, ii); the extraction of cyanide with $\mathbf{1_{SP}}$ as $\mathbf{1_{SP}}$**-CN** complex from the larger sample volume (i) and the weaker intramolecular coordination of the Dmbz base in $\mathbf{1_{SP}}$ due to the hydrophobic heterogeneous environment (ii).

The detection of cyanide with $\mathbf{1_{SP}}$ under basic conditions led to a further increase in sensitivity in accordance with experiences for the detection of cyanide under homogenous conditions. However the impact of the hydrophobic effect was less pronounced (F= 5, Table 1, entry 2 *vs* 4). At pH 9.5 concentrations as low as 0.3 mg/l of cyanide can be visually detected with $\mathbf{1_{SP}}$ (Table 1, entry 4; **Figure 2a, b**). Differences in color between immobilized $\mathbf{1_{SP}}$ and $\mathbf{1_{SP}}$**-CN** were also determined in the CIELab-system with a handheld spectrophotometer CM-2900d and have been expressed as $\Delta E$ values. Concentrations of cyanide of 1.0 and 0.3 mg/l at pH 7.5 and pH 9.5 corresponded to $\Delta E$ values of 11.4 $\pm$ 0.9 and 17.2 $\pm$ 1.7 in respect to $\mathbf{1_{SP}}$. These values were significantly above the minimum $\Delta E$ value of 2, that is correlated to obtain an univocal visual differentiation in color ($\Delta E_{vis}$).

When one of the following different anions $F^-$, $Cl^-$, $Br^-$, $I^-$, $NO_3^-$, $H_2PO_4^-$, $SO_4^{2-}$, $ClO_4^-$, $OCN^-$, $SCN^-$, $C_2O_4^{2-}$, $HCO_3^-$, $OAc^-$ (0.1 M; 5 ml; [Ches] = 20 mM, pH 9.5) or even high concentrations of halides (1 M; 5 ml; [Ches] = 20 mM, pH 9.5) were passed through the column, the red color of immobilized $\mathbf{1_{SP}}$ remained unaffected.

**Colorimetric solid phase extraction (C-SPE) of cyanide with corrinoids lacking the Dmbz base**

C-SPE was also used to detect cyanide with B12-derivatives lacking the Dmbz base at the f-side chain, which are generally called 'incomplete' corrinoids (see Scheme 2).

**Scheme 2.** Schematic representation of the coordination of cyanide to the immobilized chemosensors ($\mathbf{2_{SP}}$ – $\mathbf{4_{SP}}$; only one axial diastereomer is shown).

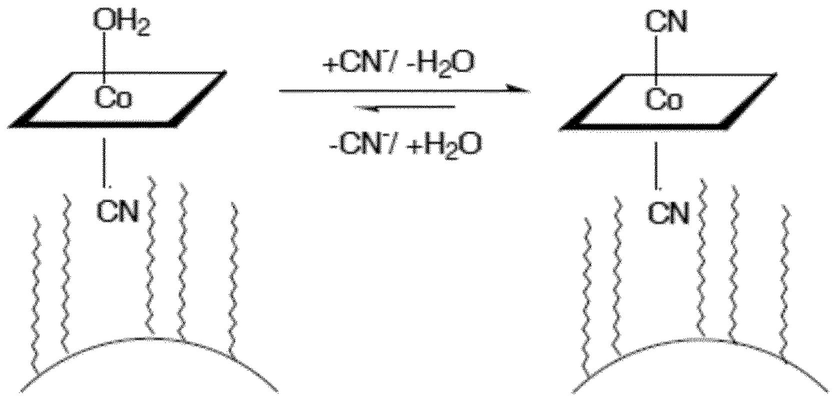


Substitution of cobalt(III)-coordinated water from the 'incomplete' corrinoid $\mathbf{2_{SP}}$ with cyanide led to the formation of $\mathbf{2_{SP}}$**-CN** as indicated by DRUV-vis spectroscopy ($\Delta\lambda_{max}$ = 45 nm, **Figure 4**). $\mathbf{2_{SP}}$ was applied to visually detect concentrations as low as 0.039 mg/l of cyanide in a 5-ml sample solution (7.5 nmol) at pH 7.5 or pH 9.5 (Table 1; entries

7, 8; **Figure 5; Figure 6**) corresponding to ΔE values of 8.0 ± 1.6 and 17.3 ± 1.3, respectively. These concentration were seven times lower than the corresponding value for the visual detection of cyanide with **2** under homogenous conditions (Table 1; entries 7 *vs* 5, 8 *vs* 6) and fell below the guideline value of 0.05 mg/l for cyanide in drinking water of the World Health Organisation (WHO). This time, the extraction and simultaneous concentration of cyanide as $\mathbf{2_{SP}}$**-CN** complex on the solid support was the main reason for the increased sensitivity of $\mathbf{2_{SP}}$ as indicated by comparable detected quantities of cyanide per quantity of chemosensors $\mathbf{2_{SP}}$ or **2** (RQ, Table 1; entries 7 *vs* 5, 8 *vs* 6). Calibration curves were generated using Kubelka-Munk transformations. **Figure 5 b**) shows the plot of the maximum of log 1/R at 583 nm of $\mathbf{2_{SP}}$**-CN** versus cyanide concentration. Quantitative determinations of cyanide are possible in the linear range between 0 and 0.075 mg/l at pH 7.5 and between 0 and 0.2 mg/l at pH 9.5. Compounds **2** and **2-CN** are susceptible for hydrolysis at the ester side chains, especially at basic pH. Therefore also compounds with partially hydrolysed side chains can be applied instead of **2.**

Chemosensor $\mathbf{2_{SP}}$ showed good, but lower selectivity for cyanide than $\mathbf{1_{SP}}$**.** Most perturbing for the visual detection of cyanide with $\mathbf{2_{SP}}$ were concentrations of 35 mg/l of $SCN^-$ (5 ml) and 640 mg/l of $I^-$ (5 ml).

Passing higher concentrated solutions (1 ml; 1 M) of $I^-$, $SCN^-$ or $OCN^-$ through $\mathbf{2_{SP}}$, led to dark violet coloured iodo-cyano ($\mathbf{2_{SP}}$**-I**), brown-violet coloured thiocyanato-cyano ($\mathbf{2_{SP}}$**-SCN**) and red colored cyanato-cyano ($\mathbf{2_{SP}}$**-OCN**) complexes. The trends in the shifts of the reflection maxima after ligand coordination to $\mathbf{2_{SP}}$ were in good agreement with the behavior of the corresponding complexes in organic solvents. Chloride was apparently not nucelophilic enough to bind to $\mathbf{2_{SP}}$. Washing the violet coloured $\mathbf{2_{SP}}$**-X** ($I^-$, $SCN^-$ or $OCN^-$) complexes with water resulted in the backformation of red coloured $\mathbf{2_{SP}}$. Compared to that, $\mathbf{2_{SP}}$**-CN** having the stronger coordinating cyanide as an axial ligand was not affected by this washing procedure. All of the following tested anions $F^-$, $Cl^-$, $Br^-$, $NO_3^-$, $H_2PO_4^-$, $SO_4^{2-}$, $ClO_4^-$, $C_2O_4^{2-}$, $HCO_3^-$, $OAc^-$ (0.1 M; 5 ml; [Ches] = 20 mM, pH 9.5) showed no interference upon passing 5 ml sample solution through the column of $\mathbf{2_{SP}}$ at pH 9.5 and pH 7.5.

**Regeneration of the immobilized chemosensors**

Regeneration of immobilized $\mathbf{1_{SP}}$**-CN**, $\mathbf{2_{SP}}$**-CN** and $\mathbf{4_{SP}}$**-CN** to $\mathbf{1_{SP}}$, $\mathbf{2_{SP}}$ and $\mathbf{4_{SP}}$, respectively had been realized by washing the filter with 0.1-1% of diluted aqueous acids (3 ml) and than with water (20 ml). The regeneration of the chemosensor is accompanied by back formation of the red color. During this process, $\mathbf{1_{SP}}$ and $\mathbf{2_{SP}}$ remained adsorbed on the top of the filter material. A series of ten subsequent runs of cyanide detection and regeneration showed no apparent loss of sensitivity or alteration of the quality of immobilized $\mathbf{1_{SP}}$ and $\mathbf{2_{SP}}$. We assume that much more consecutive tests are possible with this system.

**Applications**

Potential applications of $\mathbf{1_{SP}}$ **-** $\mathbf{4_{SP}}$ were tested for cyanide detection in liquid and gaseous phase. Endogenous cyanide liberated enzymatically from cyanogenic glycosides in samples of freshly ground cassava (*Manihot esculanta cruz*) or flax seed was detected as indicated by a colour change from red ($\mathbf{1_{SP}}$ - $\mathbf{4_{SP}}$) to violet ($\mathbf{1_{SP}}$**-CN** - $\mathbf{4_{SP}}$**-CN**) as confirmed with DRUV-Vis spectroscopy (**Figure 7**). C-SPE with B12-derivatives was also useful for the selective detection of gaseous HCN in cigarette smoke. A cigarette was connected to two C18 filter cartridges as shown in **Figure 1 b**). The first cartridge was used to remove tar from cigarette smoke. The second one contained either $\mathbf{1_{SP}}$ or $\mathbf{2_{SP}}$ for cyanide detection. After drawing cigarette smoke through the cartridges by a slight vacuum, violet coloured $\mathbf{1_{SP}}$**-CN** or $\mathbf{2_{SP}}$**-CN** with a characteristic reflection maximum at 579 nm indicated the subsequent detection of gaseous HCN (**Figure 8**).

**Individual kit for the simultaneous visual detection of cyanide and thiocyanate**

The simultaneous optical detection of anions by serial connection of SPE cartridges in C-SPE is briefly presented. This approach is distinct from parallel detection and has two major advantages (i-ii). The procedure requires less sample volume (i) and the selectivity of the immobilized chemosensor can be adjusted by consecutive extraction of potentially interfering analytes (ii). We demonstrated herein the principle of this method by the selective visual detection of mM $SCN^-$ in the presence of μM $CN^-$. In aqueous solution the visual detection of both, μM cyanide and mM thiocyanate within one sample is not possible with the corrin-based chemosenors **1-4** for two reasons (i, ii) as shown in **Figure 9.** Under these conditions, **1** is not sensitive enough to detect μM cyanide (i), whereas high concentrations of thiocyanate falsify significantly the response of **2-4** towards μM cyanide (ii). **Figure 10** represents the prototype of the C18ec material based device consisting of three filter cartridges with immobilized $\mathbf{1_{SP}}$ and $\mathbf{2_{SP}}$. Cartridge 1) contained $\mathbf{1_{SP}}$ for the selective detection of cyanide. On the top and bottom of cartridge 2) was immobilized the same chemosensor to remove and subsequently indicate the absence of excess cyanide. Cartridge 3) immobilized with sensor $\mathbf{2_{SP}}$ was used for the visual detection of $SCN^-$ in the absence of interfering cyanide.

With this strategy the optical detection of both, μM of cyanide and mM of $SCN^-$ present in a single sample was

possible. Cyanide was indicated by a color change from red (**$1_{SP}$**) to violet (**$1_{SP}$-CN**) in Filter 1) (**Figure 10**). Red coloured **$1_{SP}$** at the bottom of cartridge 2) indicated that interfering cyanide was completely removed from the sample solution. A colour change of **$2_{SP}$** from orange to brown-violet (**$2_{SP}$-SCN**; **Figure 10**) in cartridge 3) indicated the presence of $SCN^-$. Washing the cartridges with 1% of aqueous acetic acid (10 ml) led to a full recovery of the system. In principle, the simultaneous optical detection of other analytes should be possible with the flexible and modular set-up of the system.

**Comparison of C-SPE with test stripes**

As an alternative method for the rapid, semi-quantitative visual detection of cyanide, test strips impregnated with corrin-based chemosensors were developed as described in the Experimental Section. Most promising was the immobilization of **3** with seven negatively charged carboxylate side chains as **$3_{TLC}$** on Alox N/$UV_{254}$ TLC plates and of **2** with seven hydrophobic ester side chains as **$2_{TLC}$** on RP-18 HPTLC plates. Initial studies showed that the reaction of **$3_{TLC}$** to **$3_{TLC}$-CN** and **$2_{TLC}$** to **$2_{TLC}$-CN** was completed after dipping the test stripes for ten minutes into cyanide containing sample solutions. **Figure 11** shows test stripes (**$3_{TLC}$**) after being dipped for 10 min into sample solutions with increasing concentrations of cyanide (0 - 2.08 mg/l). A color change from orange to violet indicated the formation of **$3_{TLC}$-CN**. The visual detection as low as concentrations of 1 mg/l of cyanide is possible with **$3_{TLC}$**. **$2_{TLC}$** was slightly more sensitive than **$3_{TLC}$**, but still much less sensitive compared to C-SPE with a visual detection limit down to 0.04 mg/l of cyanide (**Figure 5b**).

**C-SPE with B12 and B12-derivatives immobilized on polymeric columns and discs containing C18 material**

Empore™ SPE columns (EC-SPE, 1ml, product number 4115) with C18-SD were also useful for the visual detection of cyanide. **1** and **2** were absorbed as **$1_{SP}$** and **$2_{SP}$** on the polymeric material in the Empore™ SPE columns. The visual detection limits for cyanide corresponds to the values obtained with the C18ec columns that are 0.039 mg/l of cyanide for **$2_{SP}$** and 0.26 mg/l of cyanide for **$1_{SP}$** at pH 7.5.

On the basis of these experiments cyanide was also detected using a self-made filter holder device and polymeric discs containing **$1_{SP}$** and **$2_{SP}$** on C18 filter material (EmporeTM, product number 2215) as described in the Experimental Section. The sensitivity was comparable to that with the EmporeTM SPE columns. Advantageously of this system was the manageability of the system. After passing the sample solution through the disc, the filter device can be easily opened for visual detection of cyanide by determination of the color of **$1_{SP}$** or **$2_{SP}$**. After use, the disc can be easily exchanged. Another advantage was the possibility to further reduce the amount of sensor of **$1_{SP}$** or **$2_{SP}$** from 20 nmol down to 5nmol without loss in sensitivity.

**Detection of cyanide in colored samples**

The technique of C-SPE with spatially separated extraction and detection zones was found to render possible the visual detection of cyanide in colored samples. This form of analysis is not possible in solution. The principle has been demonstrated further above for dark colored cigarette smoke and is shown here for liquid samples by identifying endogenous cyanide in samples of green colored cassava leaves (*Manihot esculenta* Crantz).

The device contains an *extraction zone* (silica C18ec column) for removal of colored interferents, before cyanide and/or hydrogen cyanide is detected with an immobilized corrinoid in the *detection zone* (**Figure 12**).

When a freshly prepared green-colored sample of a cassava leaf was passed through the device, the colored interferents remained adsorbed in the *extraction zone* (**Figure 12**)-because of attractive hydrophobic interactions with the C18 material. Endogenous cyanide was afterward indicated in the *detection zone* by a color change of **$2_{SP}$** from orange to violet (**Figure 12**). The reflection spectrum of the violet colored product with a maximum at 583 nm is coincident with the spectrum of **2-CN** under homogenous conditions (Männel-Croise, C.; Zelder, F. Inorg. Chem. 2009, 48, 1272-1274.).

**Detection of cyanide in blood**

The following methods allow the detection of cyanide in fresh blood or in (acidified) stored blood.

*Method A:* Fresh blood (100 μl) was voluntarily donated and spiked with cyanide (ImM; 10 μl). The concentration of cyanide in blood is thus 91 μM. It was subsequently dissolved in 100 μl $H_2O$ (pH 9.5, Ches 100 mM) and stored for 5 mins. Aquacyanocobyrinic acid heptamethylester (20 μl; ImM) was added and upon further 5 mins the sample was pressed through a C18ec column (1 ml). Subsequent the column was washed with 2 ml water to remove adhering blood from the C18ec material. Thereby a violet colored ring of dicyanocobester was visible in the top of C18ec material of the column (**Figure 13 b**). The diffuse reflectance spectra of the colored part of the C18ec material were recorded upon (black) passing the sample through the column (**Figure 13 a**). The spectrum of the immobilized chemosensor is given

as a reference (red) . The spectra are in agreement with the coordination of cyanide to the chemosensor.

*Method B:*

Fresh blood was obtained from the slaughterhouse of the University of Zürich and stored with sodium citrate 3.8 % to avoid coagulation. Blood (0.5 mL) was spiked with cyanide $(CN^-)_{blood}$= 0- 100 μM). 15 mins later, the blood was adjusted to pH 9.6 with Ches buffer (0.47 ml, 1M) and aquacyanocobyrinic acid heptamethylester (30 μl; 1.4 mM) was added.

After 1 min the sample was pressed through a C18ec column (1 ml) using a 10 mL syringe, connected via a Chromabond adapter PP (Macherey-Nagel) to the *C18ec* column. The column subsequently was washed with 3 ml water to remove adhering blood from the C18ec material. Thereby a red to violet colored ring of dicyanocobester was visible in the top of C18ec material of the column (Figure 1, right). The color was dependent from the concentration of $CN^-$. Different concentrations of blood cyanide can be distinguished by naked-eye or with different analytical methods before or after eluting the chemosensor-cyano complex from the solid support.

In addition to the DRUV-Vis measurements on the filter material, removing of the dicyanocobester from C18ec material is possible with methanol. Spectroscopic UV-vis measurements and thus quantification in solution can also be performed.

**Concluding remarks**

Vitamin B 12 and B 12 derivatives adsorbed on commercially available C18ec filter have been used for the straightforward and rapid detection of low concentrations of cyanide in water. In contrast to the application of the same class of chemosensors under homogenous conditions, the visual detection of cyanide below the guideline value for cyanide in drinking water of the World Health Organization (WHO; 0.05 mg/l) was possible. This method therefore improves significantly existing applications of corrinoids for the optical detection of cyanide and extends colorimetric solid-phase extraction (C-SPE) for cyanide sensing. Recently, Dai and Boon reported about the optical detection of cyanide with a heme cofactor bound in an "engineered" protein binding pocket. Reminiscent of certain 'base-off' B 12-protein complexes we present herein systems with immobilized B 12-chemosensors having a weaker intramolecular coordination of the Dmbz base to the cobalt center of the natural product. This behavior explains the up to 130-fold higher sensitivity compared to the visual detection under homogenous conditions. Applications for the selective detection of biological cyanide in food and hydrogen cyanide in cigarette smoke as well recycling of the system have been demonstrated. Furthermore, it was shown that optical detection of different analytes within a single sample was possible for a mixture of μM $CN^-$ and mM $SCN^-$ with SPE-cartridges in series connected. A distinction of these anions could not be obtained with the same class of chemosensors under homogenous conditions. In principle, the simultaneous detection of other analytes with suitable chemosensors should be possible following this approach. Applications of such easy-to-use and low-cost devices for environmental, medical and food safety control are envisaged. As an important aspect for the practical implementation of the method, the detection of cyanide and/or hydrogen cyanide in colored samples is made possible by including a clearing device for the removal of interfering colored components contained in the sample.

**Claims**

**1.** A method for selective detection of cyanide in a liquid sample, comprising determining a color change of a corrinoid compound caused upon direct contact with said sample, wherein said corrinoid compound is selected from the group consisting of:

- a cobalamine according to formula (I), wherein X is CN, $OH_2$, OH, Hal (Cl, Br, I) or $CH_3$

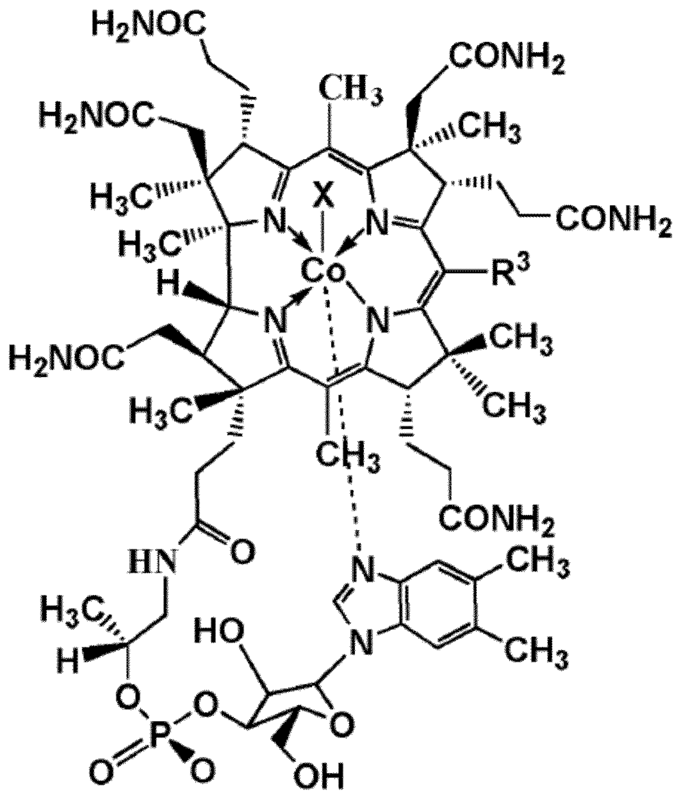

**I: X = CN, $OH_2$, OH, Hal (Cl-, Br-, I-) or $CH_3$,**
**$R^3$= H, Hal (Cl-, Br-, I-), $CF_3$, -alkyl**

- a cobyrinic acid hepta C1 -4 alkyl ester derivative (II) wherein X is CN, $OH_2$, OH, Hal (Cl, Br, I) or $CH_3$
- a cobyrinic acid (III) wherein X is CN, $OH_2$, OH, Hal (Cl, Br, I) or $CH_3$ and
- a cobinamide (IV) wherein X is CN, $OH_2$, OH, Hal (Cl, Br, I) or $CH_3$ **characterized in that** said corrinoid compound is adsorbed on a non-polar $C_4$-$C_{18}$ silica solid phase.

**2.** The method according to claim 1, wherein said solid phase is an octadecyl silica phase.

**3.** The method according to claim 1 or 2, wherein said cornnoid compound is cyanocobalamine or β-aquocobalamine or β-hydroxocobalamine.

**4.** The method according to any one of claims 1 to 3, wherein said sample is an aqueous medium containing cyanide ions.

**5.** The method according to claim 4, wherein said sample further contains thiocyanate ions and/or isocyanate ions and/or iodide ions.

**6.** The method according to any one of claims 1 to 5, wherein said detecting of cyanide comprises measurement of diffuse reflectance in the UV/visible spectral range and CIELab measurements .

**7.** The method according to any one of claims 1 to 6, further comprising the step of removing at least one interfering colored component contained in the sample.

**8.** The method according to any one of claims 1 to 4, comprising the step of forming a mixture of said liquid sample and said corrinoid compound, followed by the step of contacting said mixture with a solid phase so as to adsorb said corrinoid compound on said solid phase.

**9.** The method according to claim 8, wherein said liquid sample is a blood sample.

**10.** A kit for selective detection of cyanide in a sample, comprising:

at least one first colorimetric solid phase extraction device, said first device comprising a non-polar_$C_4$-$C_{18}$ silica solid phase loaded with a first corrinoid compound selected from the group consisting of:

- a cobalamine according to formula (I), wherein X is CN, $OH_2$, OH, Hal (Cl, Br, I) or $CH_3$
- a cobyrinic acid hepta CI -4 alkyl ester derivative (II) wherein X is CN, $OH_2$, OH, Hal (Cl, Br, I) or $CH_3$
- a cobyrinic acid (III) wherein X is CN, OH2, OH, Hal (Cl, Br, I) or CH3 and a cobinamide (IV) wherein X is CN, $OH_2$, OH, Hal (Cl, Br, I) or $CH_3$

means for passing a gaseous or liquid sample over or across said device, and means for detecting a color change of said first corrinoid compound.

**11.** The kit according to claim 10, further comprising at least one further colorimetric solid phase extraction device, said further device comprising a non-polar $C_4$-$C_{18}$ silica_solid phase loaded with a further corrinoid compound selected from the group consisting of:

- a cobalamine according to formula (I), wherein X is CN, $OH_2$, OH, Hal (Cl, Br, I) or $CH_3$
- a cobyrinic acid hepta CI -4 alkyl ester derivative (II) wherein X is CN, $OH_2$, OH, Hal (Cl, Br, I) or $CH_3$
- a cobyrinic acid (III) wherein X is CN, OH2, OH, Hal (Cl, Br, I) or CH3 and a cobinamide (IV) wherein X is CN, $OH_2$, OH, Hal (Cl, Br, I) or $CH_3$ with the provision that said first and said second corrinoid compounds are different, the kit further comprising means for passing a gaseous or liquid sample sequentially over or across said first device and said further device, and means for detecting a color change of each one of said corrinoid compounds.

**12.** The kit according to claim 10 or 11, further comprising at least one clearing device for removing interfering components contained in a liquid sample, said clearing device arranged before said first colorimetric solid phase extraction device.

**13.** Use of the kit according to claim 10 or 11 for the visual detection of cyanides in drinking water or foodstuff or blood or industrial wastewaters.

**14.** A device comprising an adsorption medium containing a corrinoid compound adsorbed on a $C_4$-$C_{18}$ silica solid phase substrate, said corrinoid compound being selected from the group consisting of:

a cobalamine according to formula (I), wherein X is CN, $OH_2$, OH, Hal (Cl, Br, I) or $CH_3$

- a cobyrinic acid hepta CI -4 alkyl ester derivative (II) wherein X is CN, $OH_2$, OH, Hal (Cl, Br, I) or $CH_3$
- a cobyrinic acid (III) wherein X is CN, $OH_2$, OH, Hal (Cl, Br, I) or $CH_3$ and
- a cobinamide (IV) wherein X is CN, $OH_2$, OH, Hal (Cl, Br, I) or $CH_3$ .

## Patentansprüche

**1.** Verfahren zum selektiven Nachweis von Cyanid in einer flüssigen Probe, umfassend die Feststellung einer durch direkten Kontakt mit der Probe verursachten Farbveränderung einer Corrinoidverbindung, wobei die Corrinoidverbindung ausgewählt ist aus der Gruppe bestehend aus:

- einem Cobalamin gemäß Formel (I), wobei X für CN, $OH_2$, OH, Hal (Cl, Br, I) oder $CH_3$ steht,

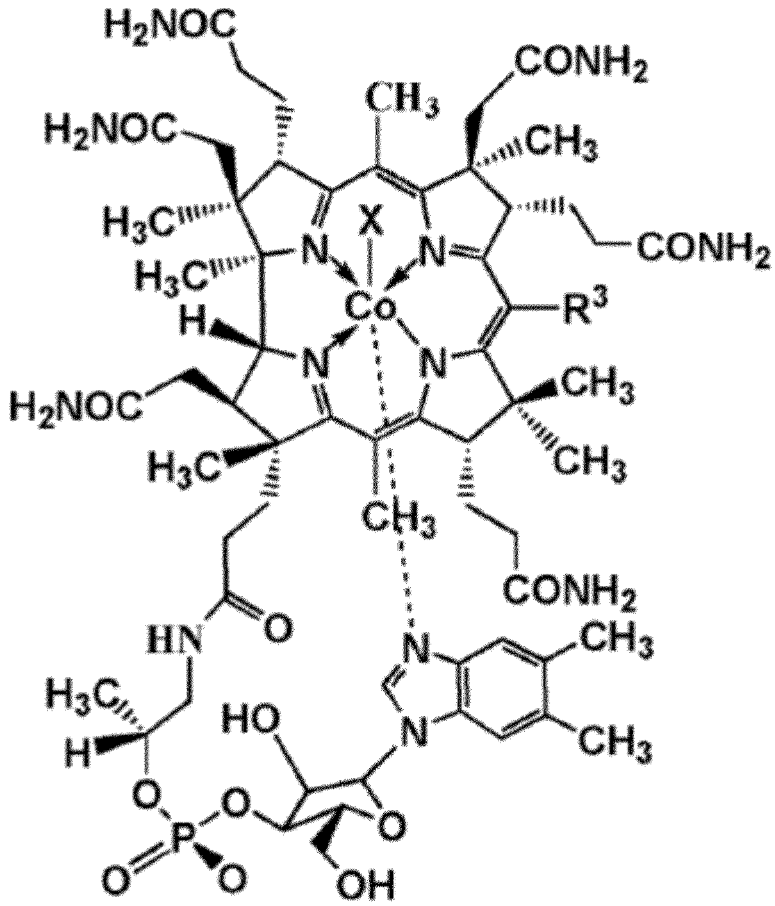

I: X = CN, $OH_2$, OH, Hal, (Cl-, Br-, I-) oder $CH_3$,
$R^3$ = H, Hal (Cl-, Br-, I-), $CF_3$, -Alkyl

- einem Cobyrinsäurehepta-C1-4-alkylesterderivat (II), wobei X für CN, $OH_2$, OH, Hal (Cl, Br, I) oder $CH_3$ steht,
- einer Cobyrinsäure (III), wobei X für CN, $OH_2$, OH, Hal (Cl, Br, I) oder $CH_3$ steht, und
- einem Cobinamid (IV), wobei X für CN, $OH_2$, OH, Hal (Cl, Br, I) oder $CH_3$ steht,

**dadurch gekennzeichnet, dass** die Corrinoidverbindung an einer unpolaren $C_4$-$C_{18}$-Siliciumdioxid-Festphase adsorbiert ist.

**2.** Verfahren nach Anspruch 1, wobei es sich bei der Festphase um eine Octadecylsiliciumdioxidphase handelt.

**3.** Verfahren nach Anspruch 1 oder 2, wobei es sich bei der Corrinoidverbindung um Cyanocobalamin oder β-Aquocobalamin oder β-Hydroxycobalamin handelt.

**4.** Verfahren nach einem der Ansprüche 1 bis 3, wobei es sich bei der Probe um ein Cyanidionen enthaltendes wässriges Medium handelt.

**5.** Verfahren nach Anspruch 4, wobei die Probe weiterhin Thiocyanationen und/oder Isocyanationen und/oder Iodidionen enthält.

**6.** Verfahren nach einem der Ansprüche 1 bis 5, wobei der Nachweis von Cyanid eine Messung des diffusen Reflexionsgrades im UV/VIS-Spektralbereich und CIELab-Messungen umfasst.

**7.** Verfahren nach einem der Ansprüche 1 bis 6, weiterhin umfassend den Schritt des Entfernens von mindestens einer in der Probe enthaltenen störenden gefärbten Komponente.

**8.** Verfahren nach einem der Ansprüche 1 bis 4, umfassend den Schritt des Bildens einer Mischung der flüssigen Probe und der Corrinoidverbindung gefolgt von dem Schritt des Inkontaktbringens der Mischung mit einer Festphase zum Adsorbieren der Corrinoidverbindung an der Festphase.

**9.** Verfahren nach Anspruch 8, wobei es sich bei der flüssigen Probe um eine Blutprobe handelt.

**10.** Kit zum selektiven Nachweis von Cyanid in einer Probe, umfassend:

mindestens eine erste kolorimetrische Festphasenextraktionsvorrichtung, wobei die erste Vorrichtung eine unpolare $C_4$-$C_{18}$-Siliciumdioxid-Festphase umfasst, die beladen ist mit einer ersten Corrinoidverbindung ausgewählt aus der Gruppe bestehend aus:

- einem Cobalamin gemäß Formel (I),
wobei X für CN, $OH_2$, OH, Hal (Cl, Br, I) oder $CH_3$ steht,
- einem Coryrinsäurehepta-C1-4-alkylesterderivat (II), wobei X für CN, $OH_2$, OH, Hal (Cl, Br, I) oder $CH_3$ steht,
- einer Cobyrinsäure (III), wobei X für CN, $OH_2$, OH, Hal (Cl, Br, I) oder $CH_3$ steht, und

einem Cobinamid (IV), wobei X für CN, $OH_2$, OH, Hal (Cl, Br, I) oder $CH_3$ steht,
Mittel zum Durchführen einer gasförmigen oder flüssigen Probe über bzw. durch die Vorrichtung und Mittel zum Nachweis einer Farbveränderung der ersten Corrinoidverbindung.

**11.** Kit nach Anspruch 10, weiterhin umfassend mindestens eine weitere kolorimetrische Festphasenextraktionsvorrichtung, wobei die weitere Vorrichtung eine unpolare $C_4$-$C_{18}$-Siliciumdioxid-Festphase umfasst, beladen mit einer weiteren Corrinoidverbindung ausgewählt aus der Gruppe bestehend aus:

einem Cobalamin gemäß Formel (I),
wobei X für CN, $OH_2$, OH, Hal (Cl, Br, I) oder $CH_3$ steht,

- einem Coryrinsäurehepta-C1-4-alkylesterderivat (II), wobei X für CN, $OH_2$, OH, Hal (Cl, Br, I) oder $CH_3$ steht,
- einer Cobyrinsäure (III), wobei X für CN, $OH_2$, OH, Hal (Cl, Br, I) oder $CH_3$ steht, und

einem Cobinamid (IV), wobei X für CN, $OH_2$, OH, Hal (Cl, Br, I) oder $CH_3$ steht,

mit der Maßgabe, dass die erste und die zweite Corrinoidverbindung voneinander verschieden sind, wobei das Kit weiterhin Mittel zum aufeinanderfolgenden Durchführen einer gasförmigen oder flüssigen Probe über bzw. durch die erste Vorrichtung und die weitere Vorrichtung und Mittel zum Nachweis einer Farbveränderung jeder dieser Corrinoidverbindungen umfasst.

**12.** Kit nach Anspruch 10 oder 11, weiterhin umfassend mindestens eine Klärvorrichtung zum Entfernen von in einer flüssigen Probe enthaltenen störenden Komponenten, wobei die Klärvorrichtung vor der ersten kolorimetrischen Festphasenextraktionsvorrichtung angeordnet ist.

**13.** Verwendung eines Kits nach Anspruch 10 oder 11 zum visuellen Nachweis von Cyaniden in Trinkwasser oder Nahrungsmitteln oder Blut oder industriellen Abwässern.

**14.** Vorrichtung, umfassend ein Adsorptionsmedium, welches eine an einem $C_4$-$C_{18}$-Siliziumdioxid-Festphasensubstrat adsorbierte Corrinoidverbindung enthält, wobei die Corrinoidverbindung ausgewählt ist aus der Gruppe bestehend aus:

einem Cobalamin gemäß Formel (I),
wobei X für CN, $OH_2$, OH, Hal (Cl, Br, I) oder $CH_3$ steht,

- einem Cobyrinsäurehepata-C1-C4-Alkylesterderivat (II), wobei X für CN, $OH_2$, OH, Hal (Cl, Br, I) oder $CH_3$ steht,
- einer Cobyrinsäure (III), wobei X für CN, $OH_2$, OH, Hal (Cl, Br, I) oder $CH_3$ steht, und
- einem Cobinamid (IV), wobei X für CN, $OH_2$, OH, Hal (Cl, Br, I) oder $CH_3$ steht.

**Revendications**

1. Procédé de détection sélective de cyanure dans un échantillon liquide, comprenant la détermination d'un changement de couleur d'un composé corrinoïde causé par le contact direct avec ledit échantillon, dans lequel ledit composé corrinoïde est choisi dans le groupe constitué de :

   - une cobalamine selon la formule (I), dans laquelle X est CN, $OH_2$, OH, Hal (Cl, Br, I) ou $CH_3$

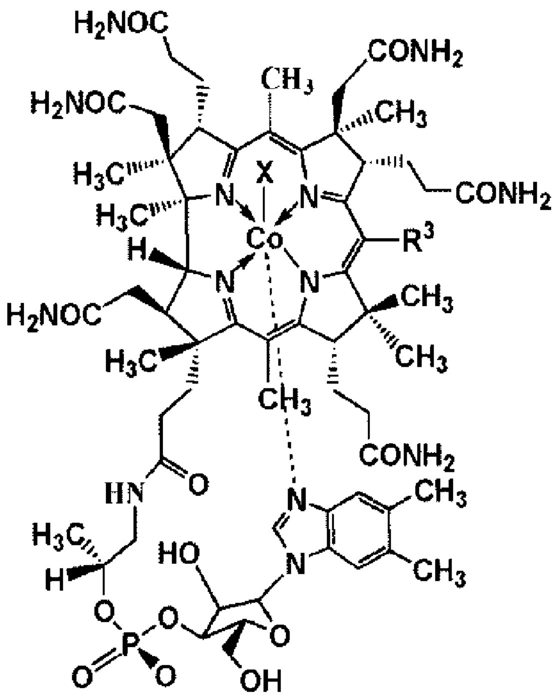

I: X = CN, $OH_2$, OH, Hal (Cl-, Br-, I-) ou $CH_3$,
$R^3$= H, Hal (Cl-, Br-, I-), $CF_3$, -alkyle

   - un dérivé hepta-ester d'alkyle en $C_{1-4}$ d'acide cobyrinique (II) dans lequel X est CN, $OH_2$, OH, Hal (Cl, Br, I) ou $CH_3$
   - un acide cobyrinique (III) dans lequel X est CN, $OH_2$, OH, Hal (Cl, Br, I) ou $CH_3$ et
   - un cobinamide (IV) dans lequel X est CN, $OH_2$, OH, Hal (Cl, Br, I) ou $CH_3$

   **caractérisé en ce que** ledit composé corrinoïde est adsorbé sur une phase solide de silice-$C_4$-$C_{18}$ non polaire.

2. Procédé selon la revendication 1, dans lequel ladite phase solide est une phase de silice-octadécyle.

3. Procédé selon la revendication 1 ou 2, dans lequel ledit composé corrinoïde est la cyanocobalamine ou la β-aquocobalamine ou la β-hydroxocobalamine.

4. Procédé selon l'une quelconque des revendications 1 à 3, dans lequel ledit échantillon est un milieu aqueux contenant des ions cyanure.

5. Procédé selon la revendication 4, dans lequel ledit échantillon contient en outre des ions thiocyanate et/ou des ions isocyanate et/ou des ions iodure.

6. Procédé selon l'une quelconque des revendications 1 à 5, dans lequel ladite détection de cyanure comprend la mesure de réflectance diffuse dans la plage spectrale UV/visible et des mesures CIELab.

7. Procédé selon l'une quelconque des revendications 1 à 6, comprenant en outre l'étape d'élimination d'au moins un composé colorant interférent contenu dans l'échantillon.

**8.** Procédé selon l'une quelconque des revendications 1 à 4, comprenant l'étape de formation d'un mélange dudit échantillon liquide et dudit composé corrinoïde, suivie phase solide de façon à adsorber ledit composé corrinoïde sur ladite phase solide.

**9.** Procédé selon la revendication 8, dans lequel ledit échantillon liquide est un échantillon de sang.

**10.** Kit de détection sélective de cyanure dans un échantillon, comprenant :

au moins un premier dispositif d'extraction en phase solide colorimétrique, ledit premier dispositif comprenant une première phase solide de silice-$C_4$-$C_{18}$ non polaire chargée avec un premier composé corrinoïde choisi dans le groupe constitué de :

- une cobalamine selon la formule (I),
dans laquelle X est CN, $OH_2$, OH, Hal (Cl, Br, I) ou $CH_3$
- un dérivé hepta-ester d'alkyle en $C_{1-4}$ d'acide cobyrinique (II) dans lequel X est CN, $OH_2$, OH, Hal (Cl, Br, I) ou $CH_3$
- un acide cobyrinique (III) dans lequel X est CN, $OH_2$, OH, Hal (Cl, Br, I) ou $CH_3$ et un cobinamide (IV) dans lequel X est CN, $OH_2$, OH, Hal (Cl, Br, I) ou $CH_3$

un moyen pour faire passer un échantillon gazeux ou liquide sur ou à travers ledit dispositif, et un moyen pour détecter un changement de couleur dudit premier composé corrinoïde.

**11.** Kit selon la revendication 10, comprenant en outre au moins un dispositif d'extraction en phase solide colorimétrique supplémentaire, ledit dispositif supplémentaire comprenant une phase solide de silice-$C_4$-$C_{18}$ non polaire chargée avec un composé corrinoïde supplémentaire choisi dans le groupe constitué de :

- une cobalamine selon la formule (I),
dans laquelle X est CN, $OH_2$, OH, Hal (Cl, Br, I) ou $CH_3$
- un dérivé hepta-ester d'alkyle en $C_{1-4}$ d'acide cobyrinique (II) dans lequel X est CN, $OH_2$, OH, Hal (Cl, Br, I) ou $CH_3$
- un acide cobyrinique (III) dans lequel X est CN, $OH_2$, OH, Hal (Cl, Br, I) ou $CH_3$ et un cobinamide (IV) dans lequel X est CN, $OH_2$, OH, Hal (Cl, Br, I) ou $CH_3$

à condition que ledit premier et ledit deuxième composés corrinoïdes soient différents, le kit comprenant en outre un moyen pour faire passer un échantillon gazeux ou liquide séquentiellement sur ou à travers ledit premier dispositif et ledit dispositif supplémentaire, et un moyen pour détecter un changement de couleur de chacun desdits composés corrinoïdes.

**12.** Kit selon la revendication 10 ou 11, comprenant en outre au moins un dispositif d'éclaircissement pour éliminer des composants interférents contenus dans un échantillon liquide, ledit dispositif d'éclaircissement étant agencé avant ledit premier dispositif d'extraction en phase solide colorimétrique.

**13.** Utilisation du kit selon la revendication 10 ou 11 pour la détection visuelle de cyanures dans de l'eau potable ou un aliment ou du sang ou des eaux usées industrielles.

**14.** Dispositif comprenant un milieu d'adsorption contenant un composé corrinoïde adsorbé sur un substrat de phase solide de silice-$C_4$-$C_{18}$, ledit composé corrinoïde étant choisi dans le groupe constitué de :

une cobalamine selon la formule (I),
dans laquelle X est CN, $OH_2$, OH, Hal (Cl, Br, I) ou $CH_3$

- un dérivé hepta-ester d'alkyle en $C_{1-4}$ d'acide cobyrinique (II) dans lequel X est CN, $OH_2$, OH, Hal (Cl, Br, I) ou $CH_3$
- un acide cobyrinique (III) dans lequel X est CN, $OH_2$, OH, Hal (Cl, Br, I) ou $CH_3$ et
- un cobinamide (IV) dans lequel X est CN, $OH_2$, OH, Hal (Cl, Br, I) ou $CH_3$.

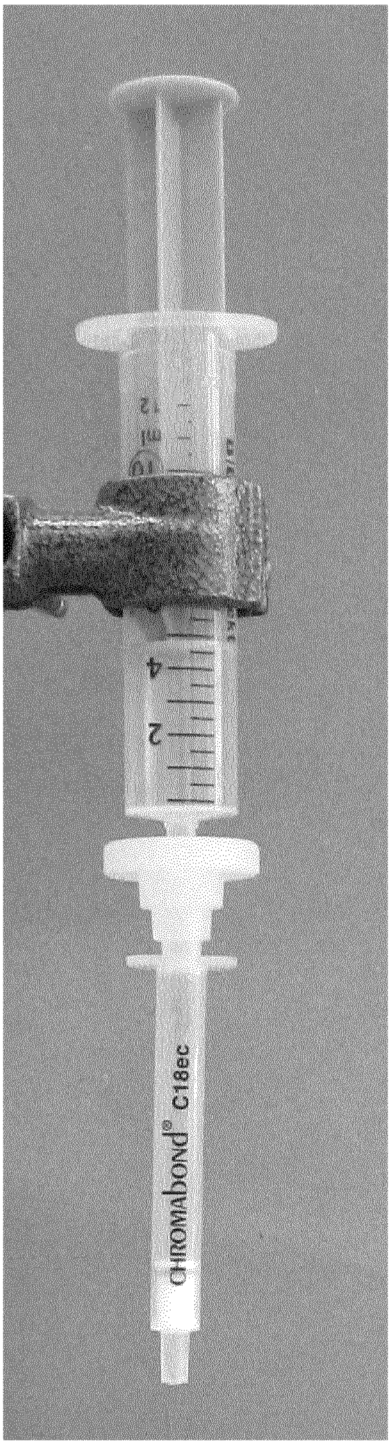


Figure 1 a)

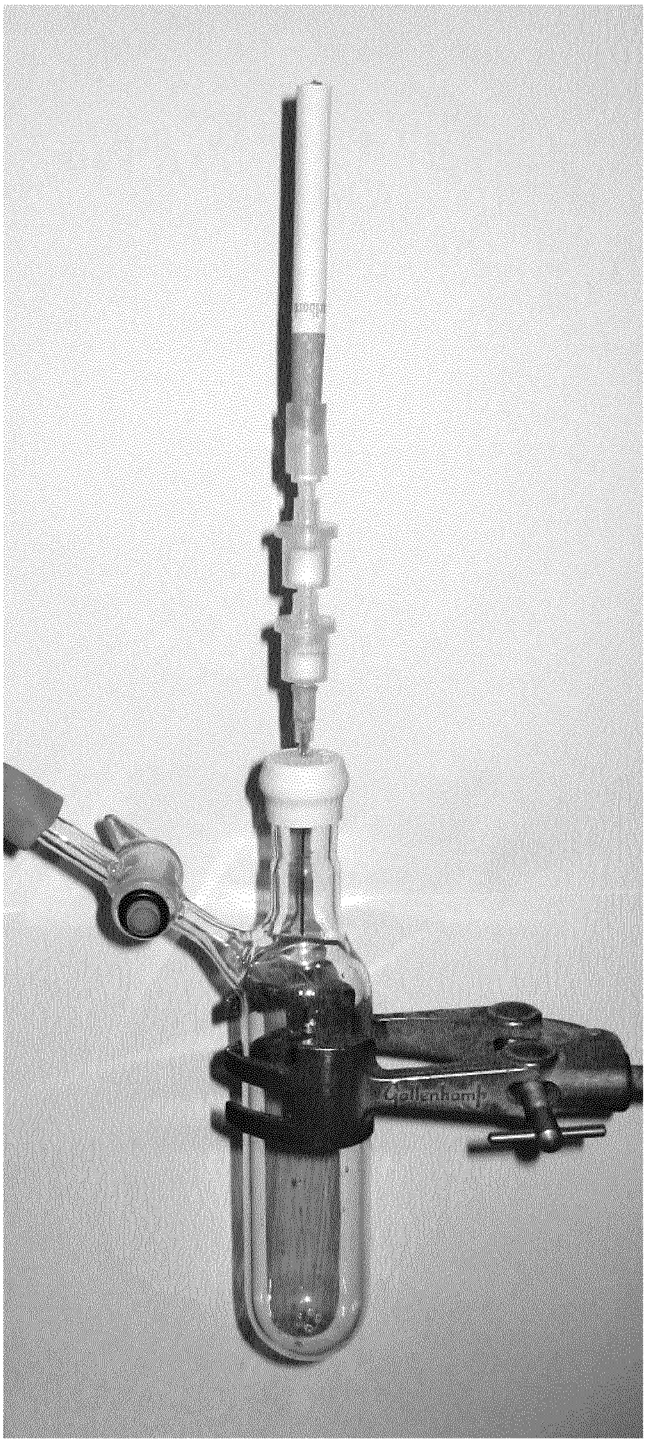

Figure 1 b)

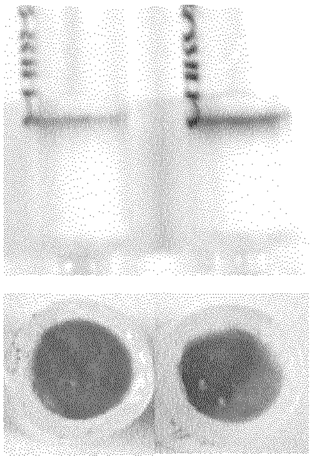

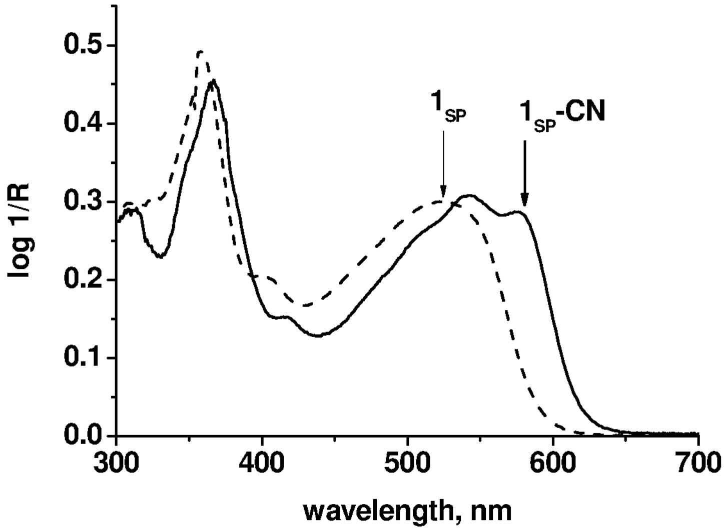


Figure 2 a)

Figure 2 b)

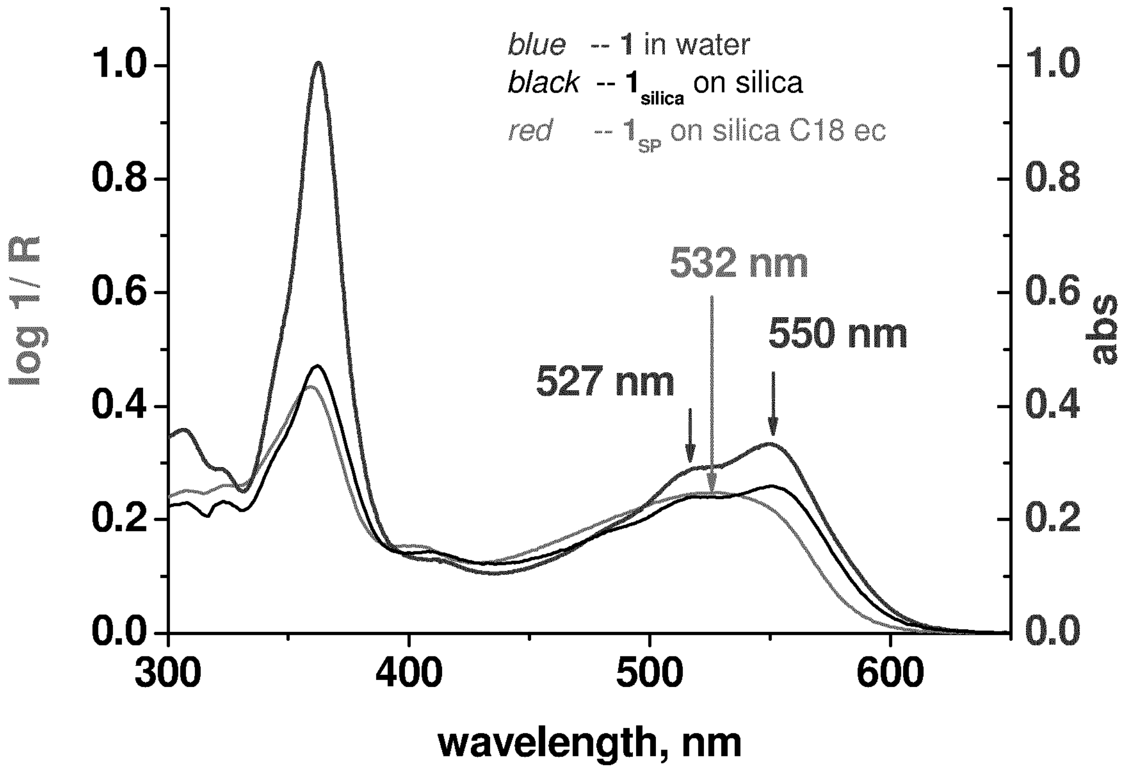


Figure 3

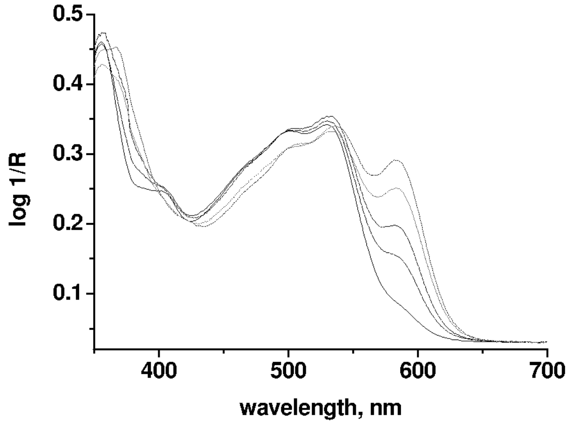


| | | | | | |
|---|---|---|---|---|---|
| L* | 69.9 | 53.3 | 51.8 | 45.5 | 44.6 |
| a* | 32.4 | 34.1 | 31.5 | 32.0 | 25.6 |
| b* | 8.1 | 3.5 | -1.5 | -7.3 | -9.3 |
| ΔE | ref. | 17.3 ± 0.2 | 20.5 ± 0.2 | 28.9 ± 0.6 | 31.4 ± 1.3 |

Figure 4 a)

Figure 4 b)

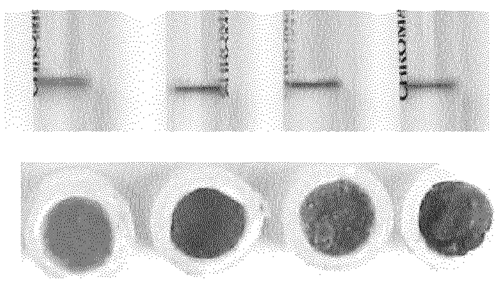

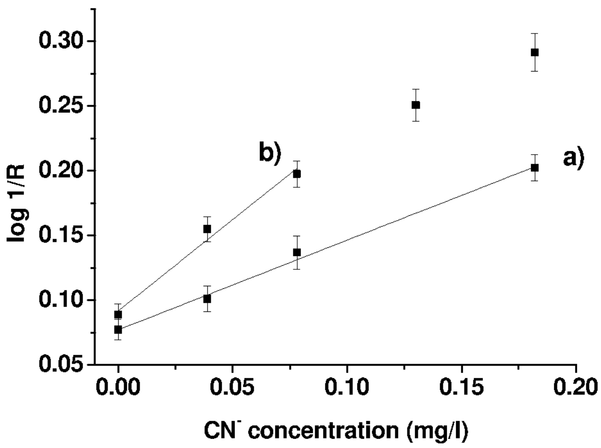


Figure 5 a)

Figure 5 b)

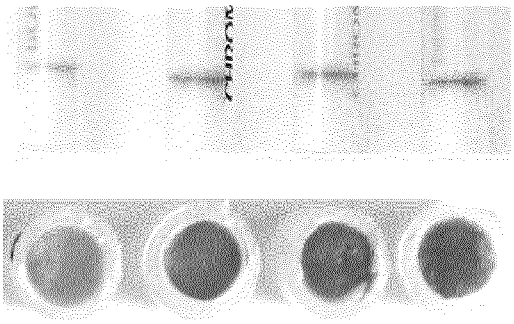

| | | | | |
|---|---|---|---|---|
| L* | 54.8 | 48.6 | 47.6 | 45.3 |
| a* | 32.3 | 35.0 | 30.1 | 29.3 |
| b* | 16.4 | 12.1 | 3.2 | 1.8 |
| ΔE | ref. | 8 ±0.2 | 15.2 ± 0.2 | 17.9 ± 0.7 |

Figure 6 a)

Figure 6 b)

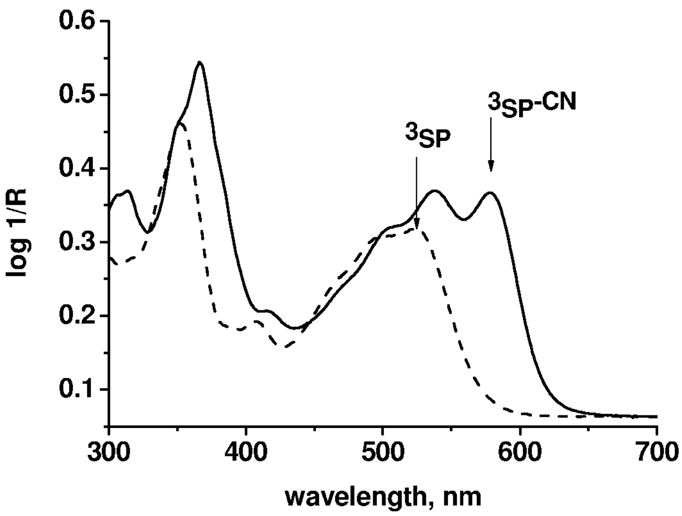


Figure 7

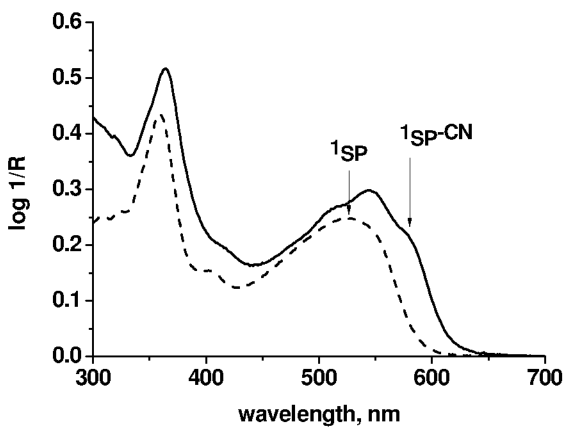


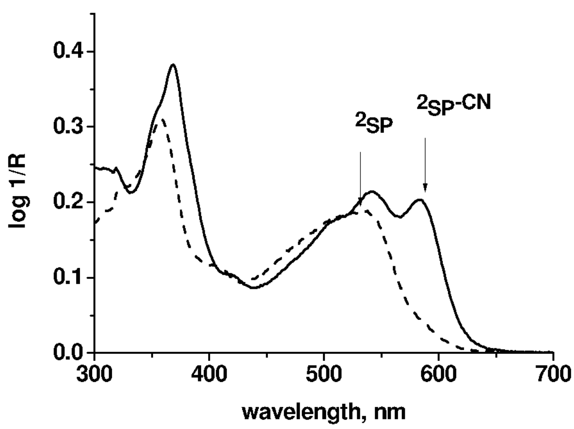


Figure 8 a)

Figure 8 b)

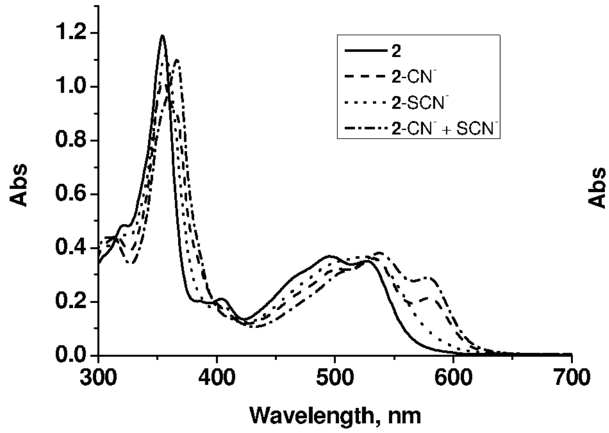


Figure 9 a)

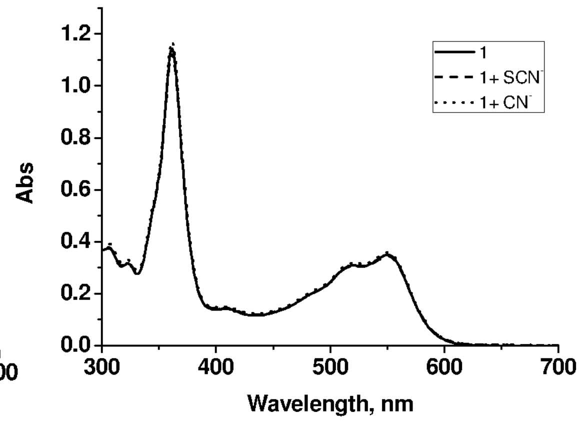


Figure 9 b)

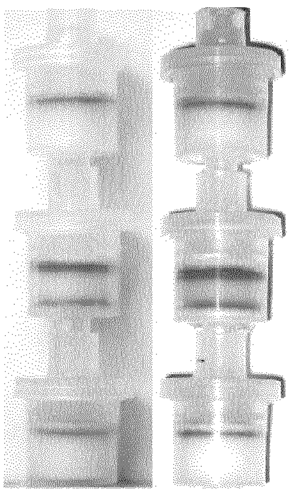

1) cyanide detection zone
color change from
red to violet

2) cyanide extraction zone

3) thiocyanate detection zone:
color change from
red to brown-violet

Figure 10

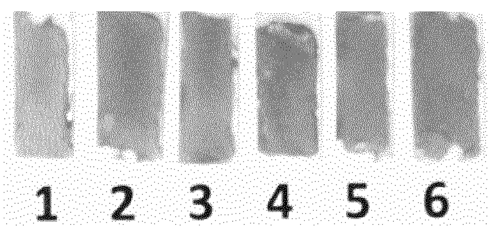


Figure 11

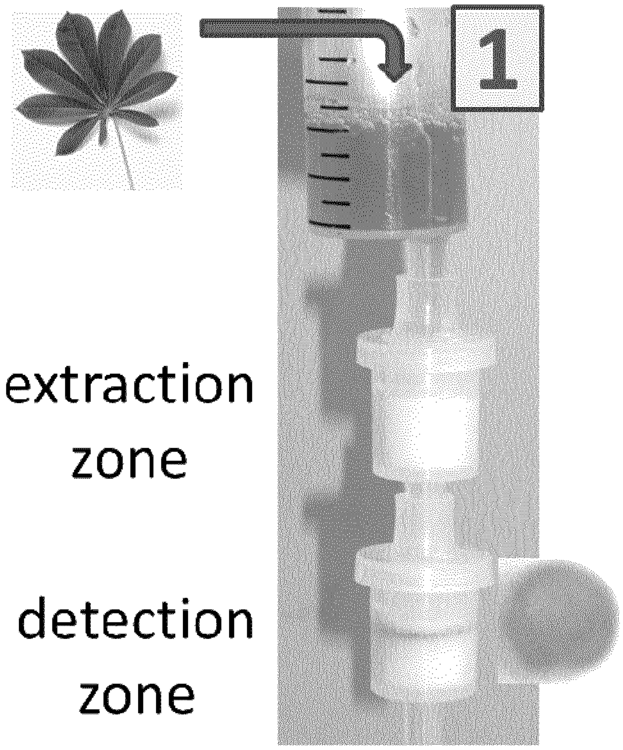


Figure 12a)

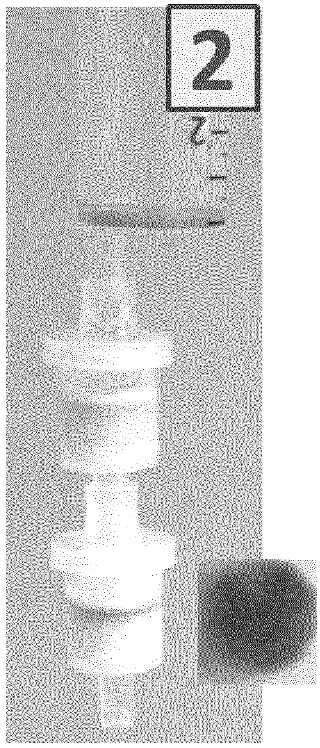


Figure 12b)

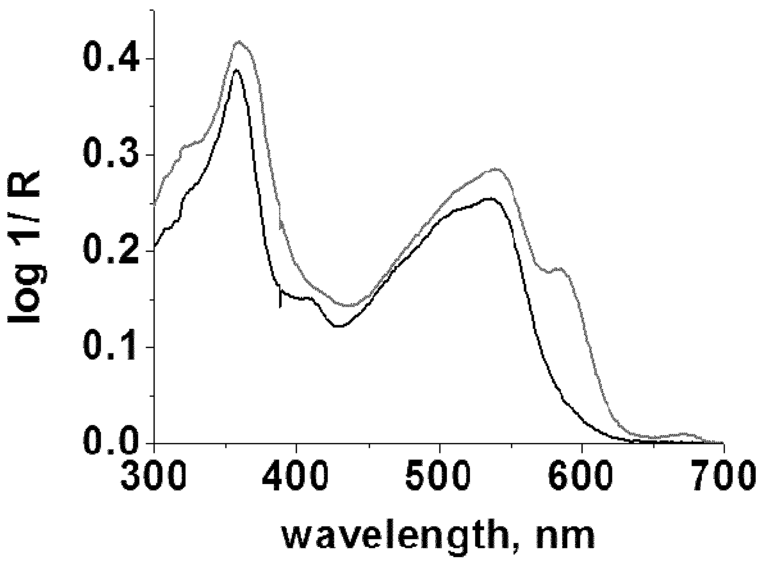


Figure 12 c)

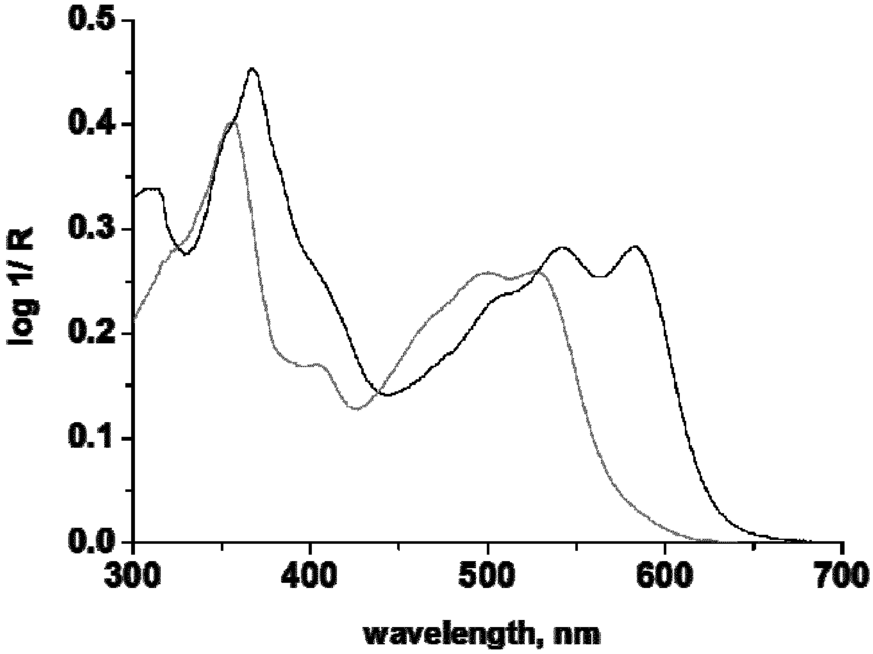


Figure 13 a)

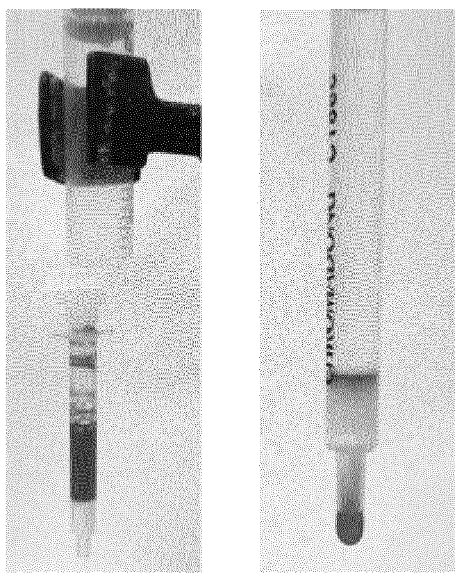

Figure 13 b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 19608808 A1


### Non-patent literature cited in the description


- **ZELDER, F. H.** *Inorg. Chem.,* 2008, vol. 47, 1264-1266
- **MÄNNEL-CROISE, C.** *Zelder, F. Inorg. Chem.,* 2009, vol. 48, 1272-1274
- **ARENA, M. P. ; PORTER, M. D. ; FRITZ, J. S.** *Anal. Chem.,* 2002, vol. 74, 185-190
- **MÄNNEL-CROISE, C. ; ZELDER, F.** *Inorg. Chem.,* 2009, vol. 48, 1272-1274